(19) Europäisches Patentamt
European Patent Office
Office européen des brevets

(11) EP 3 347 063 B1

(12) **EUROPEAN PATENT SPECIFICATION**

(45) Date of publication and mention
of the grant of the patent:
**19.11.2025 Bulletin 2025/47**

(51) International Patent Classification (IPC):
**A61L 27/34** (2006.01)  **A61L 27/36** (2006.01)
**A61L 27/58** (2006.01)  **A61F 2/24** (2006.01)

(21) Application number: **16845145.8**

(22) Date of filing: **09.09.2016**

(52) Cooperative Patent Classification (CPC):
(C-Sets available)
**A61L 27/34; A61L 27/3633;** A61L 2420/08;
A61L 2430/20                                    (Cont.)

(86) International application number:
**PCT/US2016/051005**

(87) International publication number:
**WO 2017/044787 (16.03.2017 Gazette 2017/11)**

(54) **BI-LAYER EXTRA CELLULAR MATRIX SCAFFOLDS AND USES THEREFOR**

DOPPELLAGIGE EXTRAZELLULÄRE MATRIXGERÜSTE UND VERWENDUNGEN DAFÜR

ÉCHAFAUDAGES DE MATRICE EXTRACELLULAIRE BICOUCHE ET LEURS UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.09.2015 US 201562216728 P**

(43) Date of publication of application:
**18.07.2018 Bulletin 2018/29**

(73) Proprietor: **University of Pittsburgh - of the
Commonwealth
System of Higher Education
Pittsburgh, PA 15260 (US)**

(72) Inventors:
• **BADYLAK, Stephen, F.**
**West Lafayette, Indiana 47906 (US)**
• **D'AMORE, Antonio**
**Pittsburgh, Pennsylvania 15206 (US)**
• **WOLF, Matthew, T.**
**McKees Rocks, Pennsylvania 15136 (US)**
• **WAGNER, William, R.**
**Gibsonia, Pennsylvania 15044 (US)**

(74) Representative: **Kraus & Lederer PartGmbB
Thomas-Wimmer-Ring 15
80539 München (DE)**

(56) References cited:
US-A- 4 801 299      US-A1- 2009 142 836
US-A1- 2011 224 800  US-A1- 2013 253 663
US-A1- 2014 099 330  US-A1- 2014 100 648
US-A1- 2014 309 739  US-A1- 2015 246 157
US-B1- 6 428 802

• GUOXU ZHAO ET AL: "Recent Advances in
Electrospun Nanofibrous Scaffolds for Cardiac
Tissue Engineering", ADVANCED FUNCTIONAL
MATERIALS, vol. 25, no. 36, 1 September 2015
(2015-09-01), Hoboken, USA, pages 5726 - 5738,
XP055644188, ISSN: 1616-301X, DOI: 10.1002/
adfm.201502142

Remarks:
The file contains technical information submitted
after the application was filed and not included in this
specification

EP 3 347 063 B1

(52) Cooperative Patent Classification (CPC): (Cont.)

C-Sets
**A61L 27/34, C08L 75/04;**
**A61L 27/34, C08L 89/00**

**Description**

**CROSS REFERENCE TO RELATED APPLICATIONS**

This application claims the benefit of United States Provisional Patent Application No. 62/216,728, filed September 10, 2015.

Provided herein is a bilayer medical device (e.g., "patch") for use in treating ischemic cardiomyopathy as well as for treatment of other conditions. Methods of making the device also are provided herein.

Remodeling of the left ventricle (LV) is a compensatory response that can be initiated following myocardial infarction, where wall thinning, chamber dilation and shape modification occur to maintain cardiac output by increasing the stroke volume. Approaches developed to mitigate LV remodeling include drug treatments (e.g., beta blockers + acetyl choline esterase inhibitors), surgical procedures, and epicardial restraint devices. While these approaches have met with success in some patient populations, progression towards end-stage heart failure still occurs. Epicardial restraint devices, meant to provide a physical block of continued cardiac dilatation, have the disadvantage of encapsulating the heart in a permanent foreign body. While cell injection-based therapies have been proposed as a strategy to treat the initial stage (<1 wk) of cardiac ischemia, which is characterized by heavy cardiomyocyte loss, clinical trials in acute myocardial infarction have largely not been positive. More than nine studies have completed phase II-III clinical trials, but meta-analysis of cell based therapies for acute myocardium infarction has shown minimal-to-no benefit in terms of clinical outcomes and LV function. In contrast, similar approaches exhibited more encouraging results when applied to heart failure. Meta-analysis of cell therapy trials for patients affected by heart failure have shown that cell treatment can potentially reduce both the risk of mortality and re-hospitalization. With respect to cells delivered in the context of a scaffold, data are mostly in pre-clinical models, yet results have shown increased cell efficacy and viability in the presence of a scaffolding material.

A promising alternative to mitigate LV dilatation that is increasingly gaining attention, is to employ a biodegradable cardiac patch. Such patches are generally applied to the epicardium to provide temporary local mechanical support and impact the ventricle maladaptive remodeling to help sustain LV function. More than twenty *in vivo* studies focusing on cardiac patch have been reported, with the dominant research paradigms including: cellularized patches providing putative paracrinal regenerative activity, ventricular mechanical support, the controlled release of bioactive molecules from acellular scaffolds, pre-vascularized patches, scaffold degradation potentially impacting the temporal sequence of the inflammatory response, and patch structural organization to mitigate electrical uncoupling. In spite of the apparent diversity in research approaches, there is a need for devices that provide adequate and appropriate mechanical support and which augment cellular engraftment. Such devices are previously unavailable. Likewise, superior products are needed for repair of other anatomical structures, such as: other cardiac structures, including the right ventricular outflow tract, epicardial, or endocardial repair; aorta repair; large blood vessel repair, such as repair of the inferior vena cava; skeletal muscle repair; abdominal wall repair, gastrointestinal repair, such as esophageal, gastroesophageal, or intestinal repair. Guoxu Zhao et al. discloses in "Recent Advances in Electrospun Nanofibrous Scaffolds for Cardiac Tissue Engineering" (Adv. Funct. Mat. 2015, 25, 5726-5738) the development of nanofibrous materials fabricated by electrospinning as desirable scaffolds for tissue engineering applications because of the mimicking structure of protein fibers in native ECM.

**SUMMARY**

A cardiac patch is useful in tissue repair, such as for repair of an infarct in a heart. The cardiac patch of the invention comprises a first layer comprising a network of synthetic, biocompatible, bioerodible fibers and an aqueous solution dispersed throughout the network of synthetic, biocompatible, bioerodible fibers of the first layer, and a second layer comprising a network of synthetic, biocompatible, bioerodible fibers, attached to the first layer, and comprising acid protease-solubilized ECM dispersed throughout the network of synthetic, biocompatible fibers of the second layer, wherein the fibers of the first layer are arranged anisotropically, wherein the layers are fibrous electrospun layers, and the method comprises implanting said cardiac patch at or adjacent to the tissue damage or defect such that the second layer contacts the damaged or defective cardiac or cardiovascular tissue.

Also provided, but not claimed, is a method of preparing a cardiac patch comprising depositing on a substrate, a network of synthetic, biocompatible fibers to form a first layer and depositing a second layer comprising an ECM material to form a device comprising a first polymeric layer comprising a network of synthetic, biocompatible fibers; and a second layer attached to the first layer comprising an ECM material. The synthetic, biocompatible fibers are optionally elastomeric. In one aspect, no cells are deposited on or in the device prior to use. In another aspect, the first layer is deposited on the substrate and the second layer is deposited over the deposited first layer. The term "over," when describing the relative position or deposition of two layers in the device and methods described herein, unless specifically noted, does not imply complete coverage, but means coverage of at least a portion of the layer described relative to another layer, it also does not imply any spatial orientation of the device (e.g., it does not imply up or down relative to Earth's gravity or relative to a patient, manufacturer or user of the device), and further does not exclude, unless direct contact is indicated, deposition, inclusion, or otherwise introduction of one or more additional layers or compositions between the described layers. In another aspect, the second layer is deposited on the substrate and the first layer is deposited over the deposited second layer. In yet another aspect, the method comprises concurrently depositing a network of synthetic, biocompatible fibers with the ECM material to form the second layer, and optionally wherein the fibers of the second layer are deposited isotropically, and optionally the fibers of the first layer are deposited anisotropically.

Also provided, but not claimed, is a method of treating damage or a defect in a tissue in a patient, comprising implanting the device according to any aspect described herein, at or adjacent to the tissue damage or defect. In one aspect, the tissue is cardiac or cardiovascular tissue. In another aspect, the damage or defect is: damage resulting from a myocardial infarction; in a right ventricular outflow tract of a heart; damage resulting from a myocardial infarction, such as an infarct; is in a heart valve; is in the aorta or vena cava.

The references to the methods of treatment by therapy or surgery in this description are to be interpreted as references to the cardiac patches of the present invention for use in those methods.

## BRIEF DESCRIPTION OF THE DRAWINGS

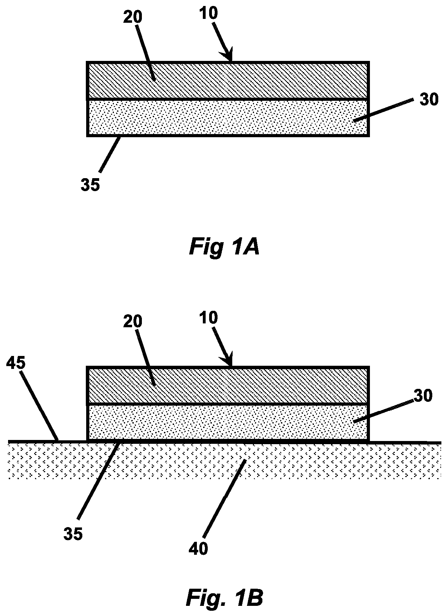

Figures 1A and 1B are schematic diagrams of a device as described herein.

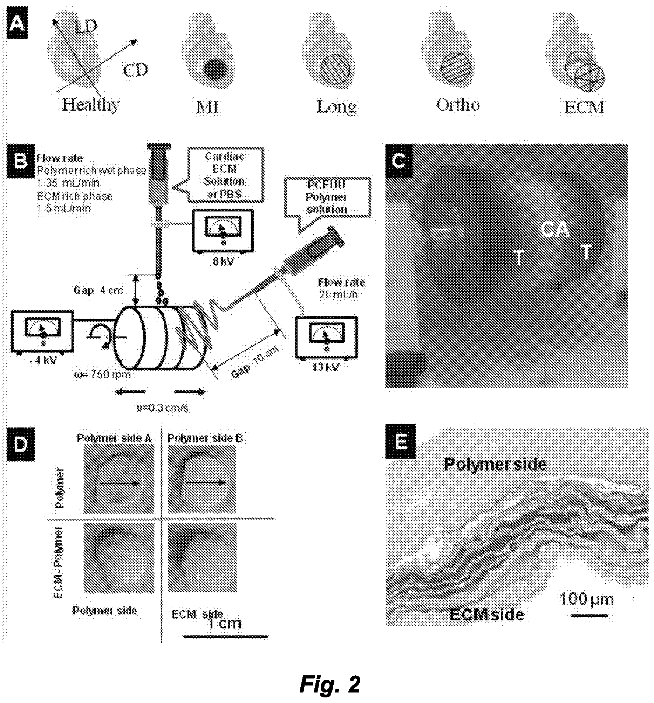

Figure 2. Bi-layer scaffold fabrication. A) control and implanted patches groups graphical representation, from left to right: healthy and MI control groups, longitudinal, orthogonal and ECM groups. B) Fabrication configuration for bi-layer "open-face sandwich" biohybrid scaffold. C) ECM/polymer collecting area (CA) and insulating tape (T) focusing the material deposition on a localized region of the rotating mandrel. D) Cardiac patch samples: polymer only (top row) and the bi-layer patch (bottom row). E) Masson's staining for bi-layer scaffold patch cross-section showing both polymer and ECM rich layers.

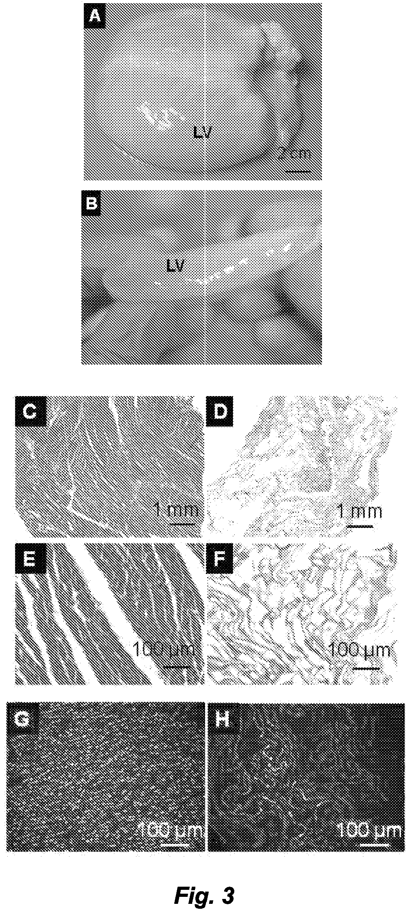

Figure 3. Whole heart decellularization. A-B) Whole porcine heart after successful decellularization; C-F) H&E stained LV cross sections of intact (C, E) and decellularized porcine heart (D, F) showing absence of cell nuclei and preserved ECM structure; G - H) DAPI staining (blue in original) of LV cross sections before and after the decellularization process confirming absence of cell nuclei, tissue and collagen auto-fluorescence signal was intentionally acquired and is shown in red (red in original).

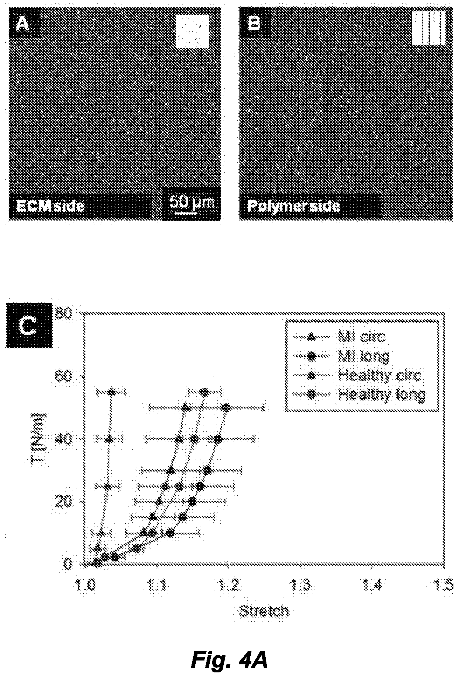

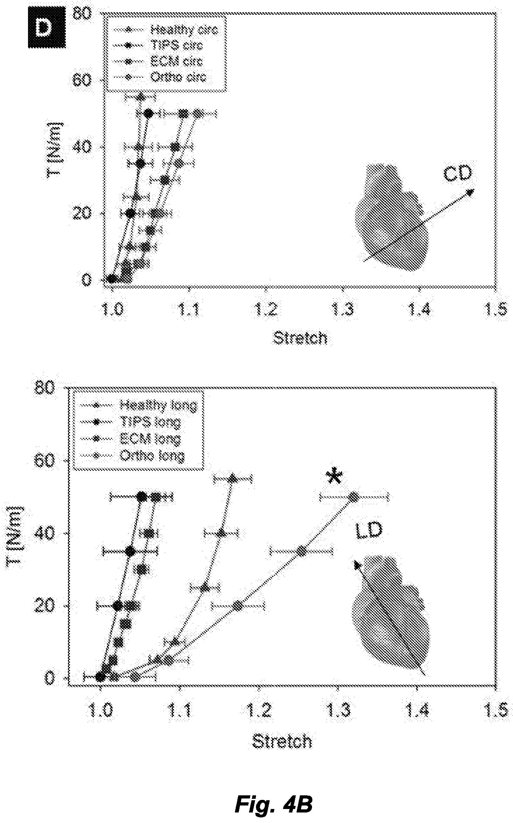

Figure 4. Bi-layer scaffold structure characterized by multi-photon imaging and biaxial mechanical testing. A) ECM rich layer multi-photon image stack (500x500x100 $\mu m^3$) showing polymer fibers randomly distributed B) Polymer rich layer multi photon image stack showing highly aligned polymer, C) Equi-membrane Lagrangian tension (T) biaxial mechanical testing of healthy and infarcted myocardial samples 2 wk post ligation procedure. D) Equi-membrane tension biaxial response for healthy LV wall and pre-implanted scaffolds. $n \geq 5$, mean $\pm$ sem, *$p<0.05$ vs. other groups.

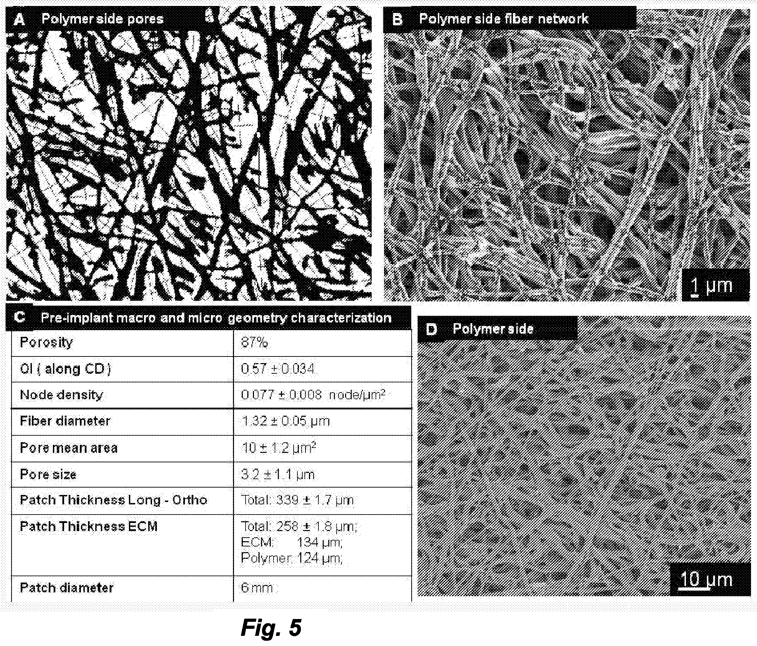

Figure 5. SEM based digital image analysis of single layer pre-implant scaffolds. A) representative image of scaffold pores after digital processing B) representative image of detected scaffold fiber network after and before C) scaffold micro-architecture features and macroscopic geometry D) digital processing (n=6 samples, 7 images for each sample, mean $\pm$ sem).

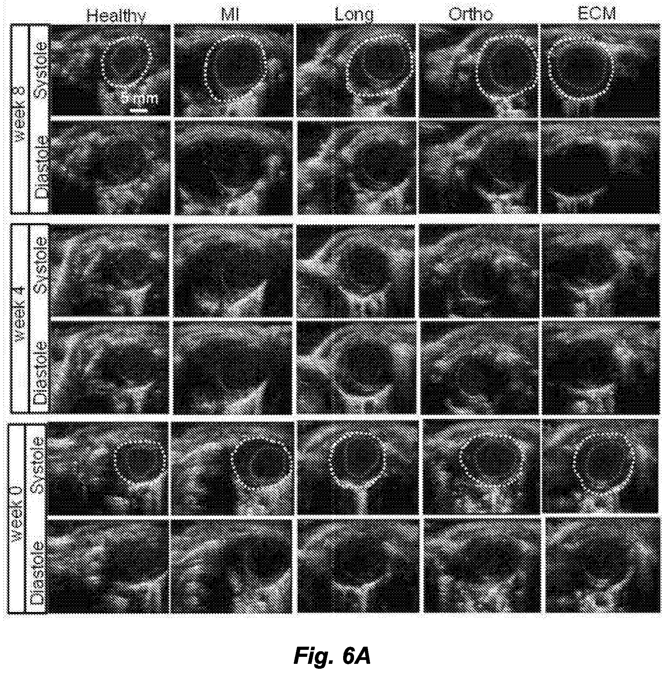

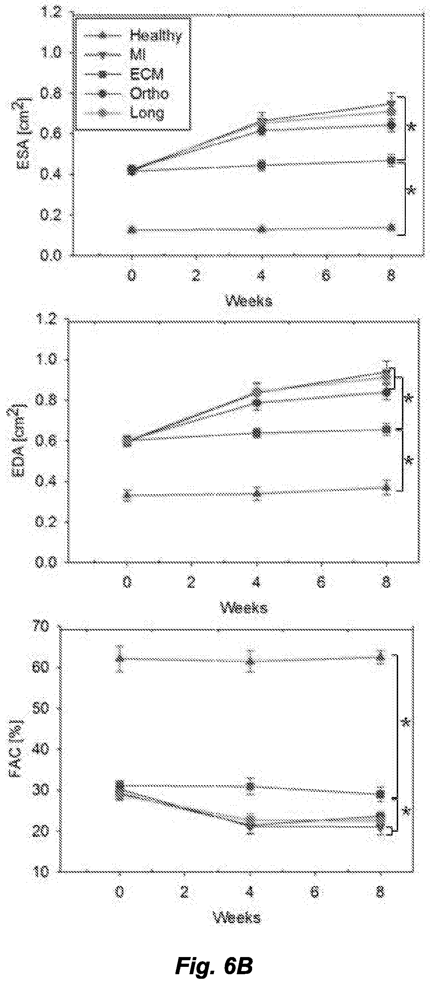

Figures 6A and 6B. Echocardiographic assessment: (6A) Representative echocardiographic images of cardiac cross sections in systole and diastole at 0, 4 and 8 week time-points. Endocardial side (red dotted edges in original) and epicardial side (white dotted edges in original) are highlighted for the 0 and 8 week time-points in systole. (6B) End systolic area (ESA), end diastolic area (EDA) and fractional area change (FAC) after patch placement (week 0 is 2 week post-MI) and at 4, 8 week for rats implanted with a PECUU control patch (Long, Ortho), bi-layered ECM patch or control groups with MI alone, or no MI (healthy). N=6, mean $\pm$ sem, *$p<0.05$.

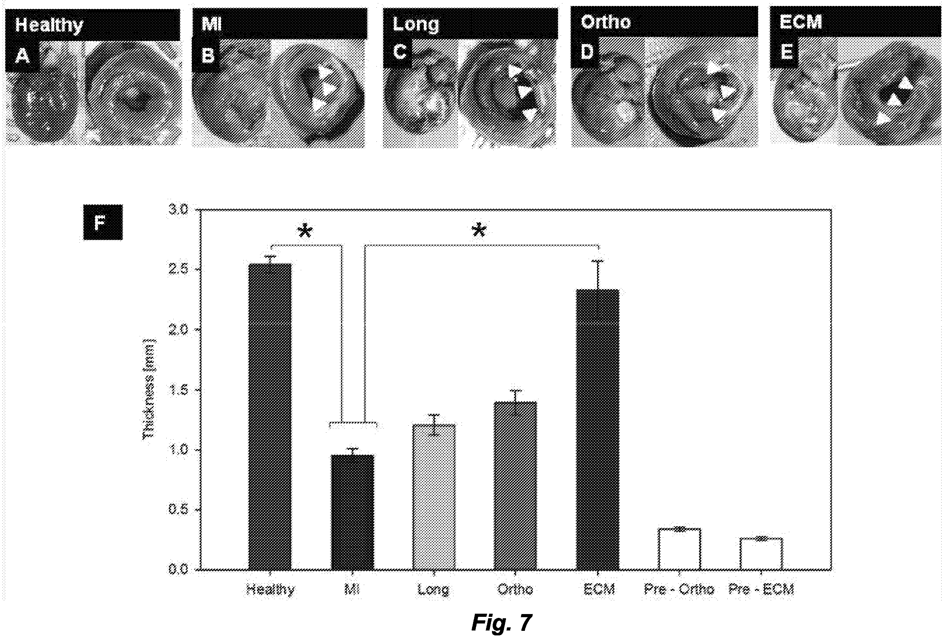

Figure 7. LV wall thickness at 8 wk. Control groups (A-B), and patched hearts (C-E) 8 week after patch placement (10 week post-MI). Mean LV wall thickness (F) was higher when the ECM bi-layer patch was utilized. Pre-Ortho and Pre-ECM represent patch thickness prior to implantation. n=6, mean + sem, *$p<0.05$, white arrows indicate implantation site.

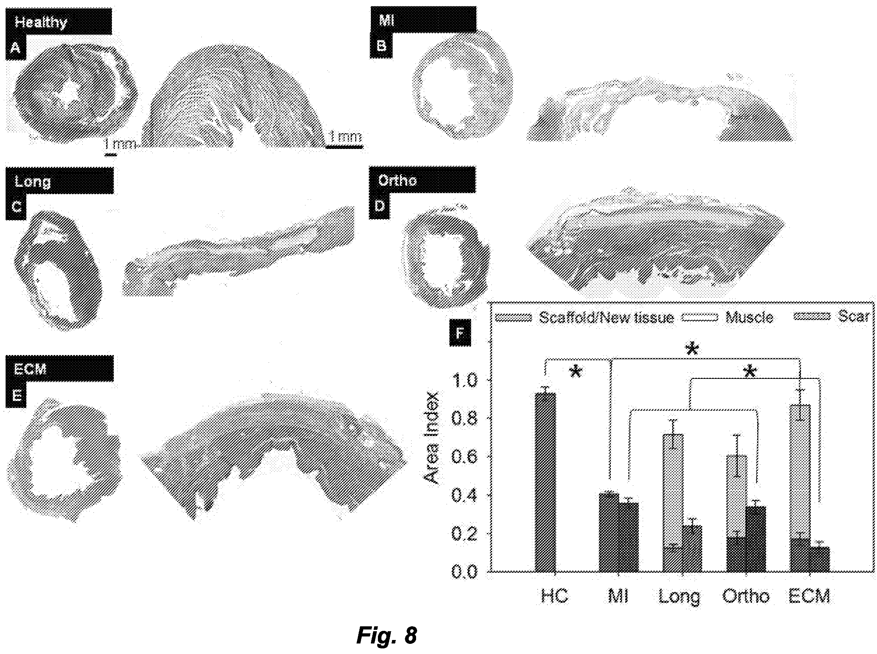

Figure 8. MT staining, muscle and scar area index. (A-E) Typical MT stained whole heart sections and infarct/patch regions at the 8 wk time point. The ECM scaffold explants showed higher host cell infiltration than either of the oriented PECUU patches. (F) Quantitative comparison of muscle, scaffold/newly formed tissue and scar areas in histological sections between patch types. n=6, mean $\pm$ sem, *$p<0.05$.

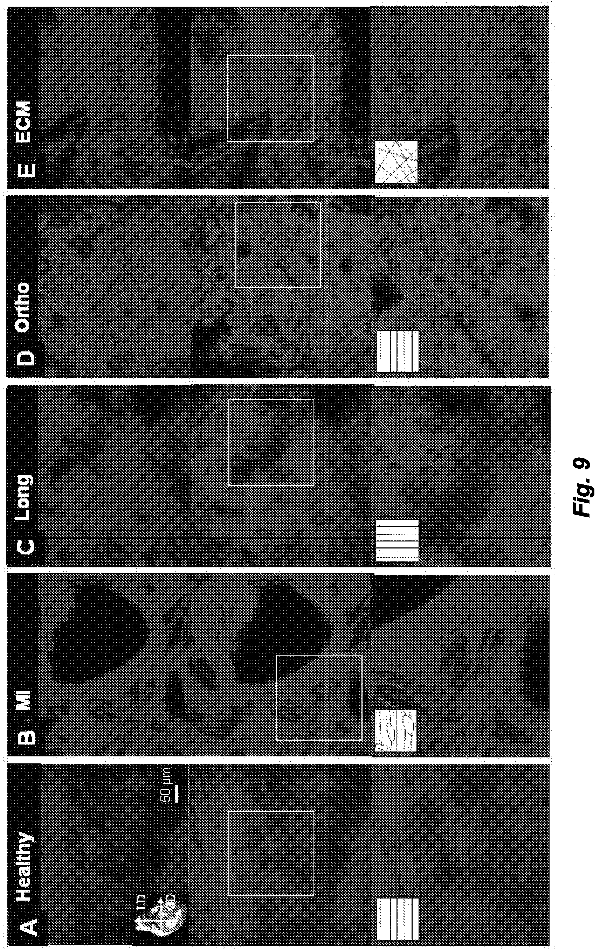

Figure 9. Multi photon explants and control group analysis. A) intact collagen network from the healthy heart, circumferentially oriented, B) damaged collagen fiber network from hearts 8 wk after MI, C) longitudinally aligned scaffold fibers (blue in original) and de-novo collagen fiber network (red in original) for the longitudinally oriented PECUU patch explanted at 8 wk. D) orthogonally/circumferentially aligned scaffold fibers (blue in original) and de-novo collagen fiber network (red in original) for the orthogonally oriented PECUU patch explanted at 8 wk. E) ECM bi-layered patched hearts explanted at 8 wk showed randomly oriented collagen and scaffold fibers.

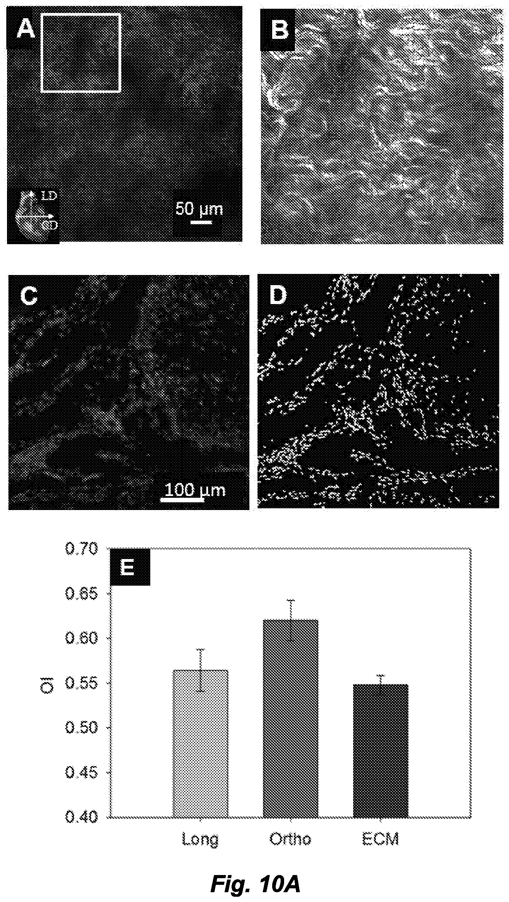

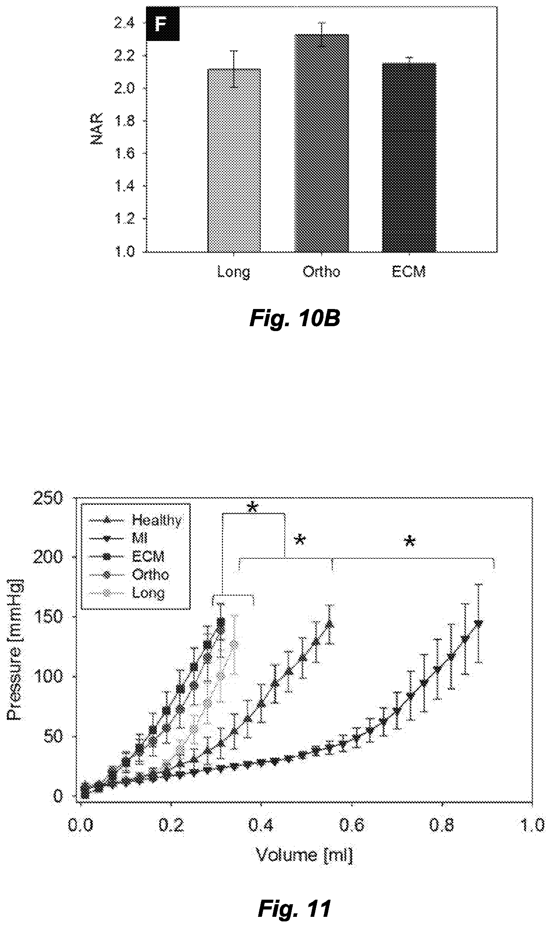

Figure 10. De novo ECM fiber network orientation index (OI) and infiltrating cell nuclear aspect ratio (NAR). A) representative multi-photon image stack for patch explants at 8 weeks showing collagen second harmonic generation. B) digital image analysis detected fibers in the white square indicated in A, OI was calculated based on this information. C) representative fluorescence image of patch explants at 8 weeks with DAPI staining to detect cell nuclei. D) cell nuclei identified by image segmentation. E) OIs for patch explants. F) NARs for patch explants, (n≥5 mean ± sem).

Figure 11. Ex-vivo LV pressure-volume testing (n≥6 mean ± sem; *p<0.05) showing increased compliance for the ischemic ventricle as well as the LV chamber stiffening effect produced by the cardiac patches.

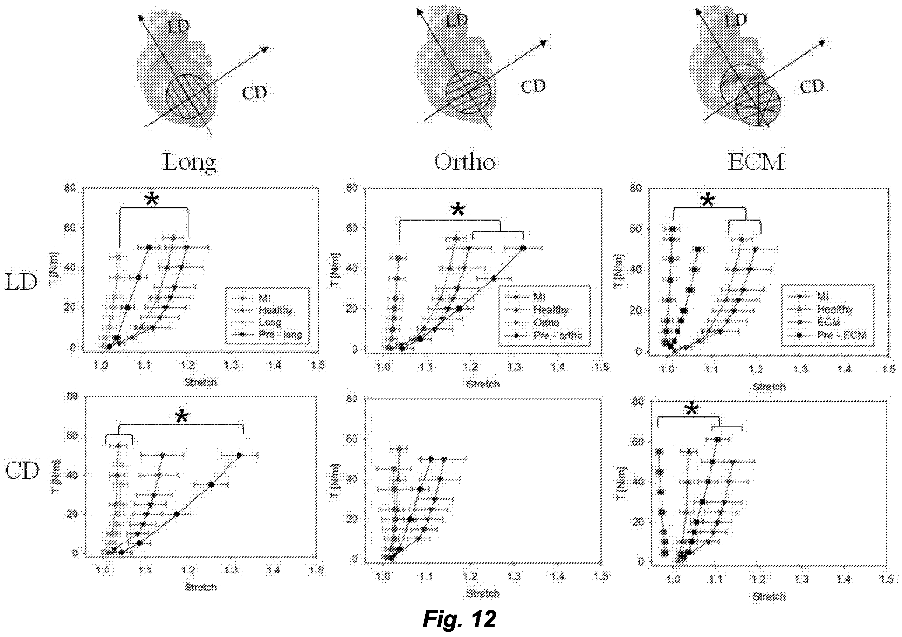

Figure 12. Biaxial mechanical characterization of the patched LV wall. Equi-membrane tension protocol applied 8 wk after patch placement. In the top row stretch is measured in the longitudinal direction (LD), while the bottom row presents stretch in the circumferential direction (see diagrams above the graphs). Control groups are 10 wk post-MI and healthy hearts. n≥5, mean ± sem, *p<0.05.

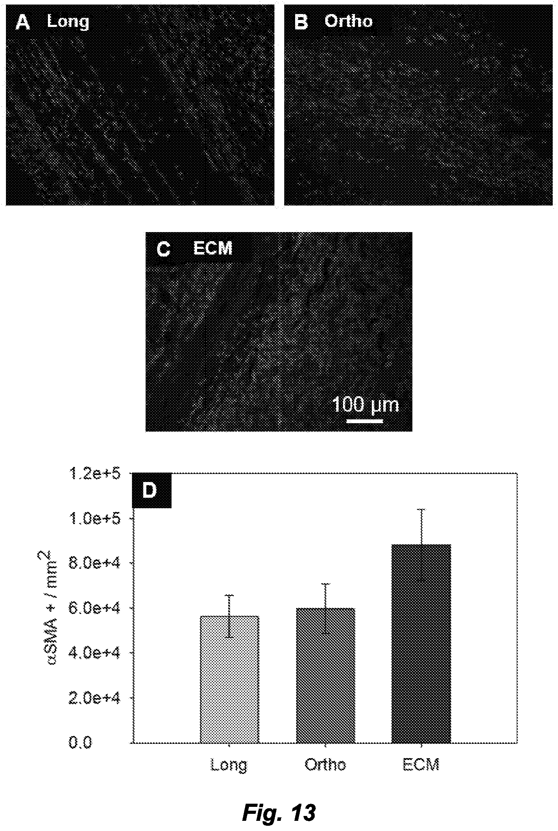

Figure 13. Immunofluorescence assessment of αSMA. αSMA positive (red in original) and DAPI (blue in original) stained sections for longitudinally (A), and orthogonally (B) oriented PECUU patches, and for the bi-layered ECM patch (C) at 8 wk post-placement. (n≥5 mean ± sem). D) αSMA/mm$^2$ positive pixels vs. groups.

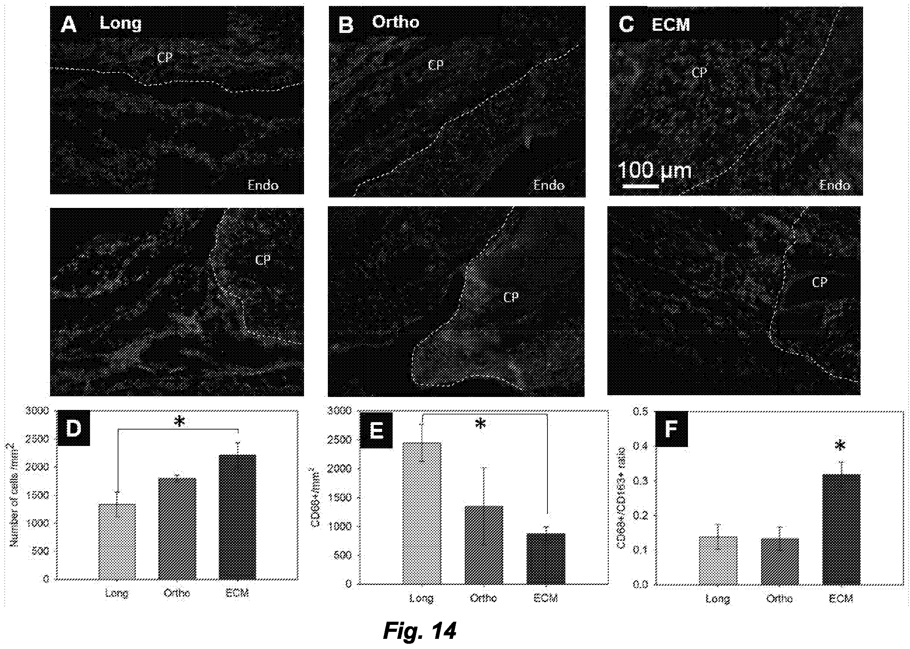

Figure 14. Immunofluorescence assessment for infiltrating macrophages at 8 wk. CD68 CD163 and DAPI staining for the longitudinally (A) and orthogonally (B) oriented PECUU patches and for the bi-layered ECM patch (C) (n≥5 mean ± sem. *p<0.05). (D) DAPI staining showed greater host cell infiltration and (E) lower CD68+ cell density for the ECM cardiac patch explants versus longitudinally oriented PECUU patches. F) The M2 CD163+/CD68+ ratio was significantly higher in bi-layered ECM cardiac patch explants than either PECUU patch type.

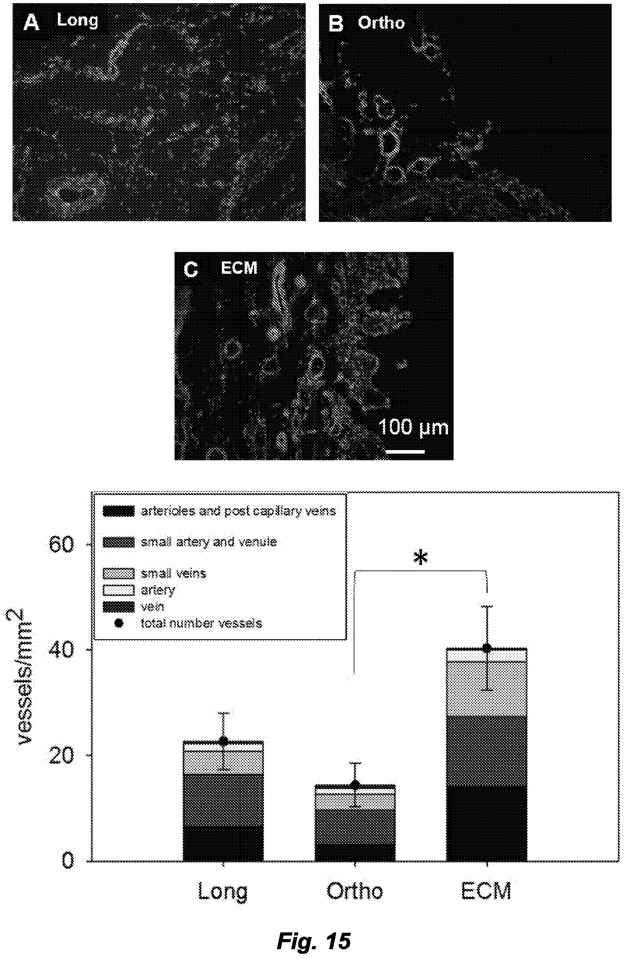

Figure 15. Immunofluorescence assessment for vascularization at 8 wk. CD31 (green in original), αSMA (red in original) and DAPI (blue in original) staining for the longitudinally (A) and orthogonally (B) oriented PECUU patches, and for the bi-layered ECM patch (C) (n≥5 mean ± sem; *p<0.05 for total vessel number). Vessels were identified by CD31 and αSMA co-localization, vessel type was determined based on size, intima-media layer thickness ratio, shape, presence/absence of intimal layer.

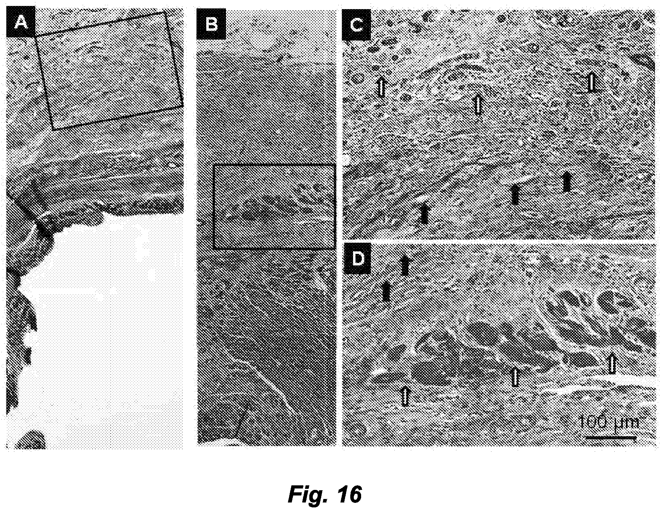

Figure 16. MT staining, details of bi-layer cardiac patch. (A), (C) representative slide of bi-layer patch explants at 8 wk showing functional vessels formation at the scaffold tissue interface, white and black arrows highlight blood vessels and scaffold fibers respectively. (B), (D) representative slide showing preserved cardiac muscle underneath the patch area, white and black arrows highlight putative cardiac muscle and scaffold fibers respectively.

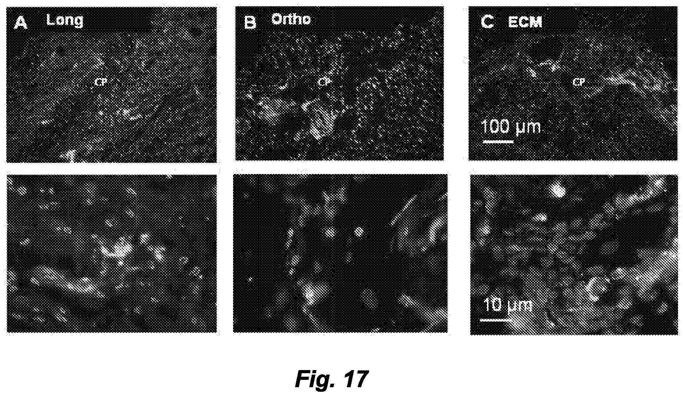

Figure 17. Immunofluorescence assessment for pericytes and their association with vascular structures at 8 wk. CP (cardiac patch region), vWf (green in original), NG2 (red in original) and DAPI (blue in original) staining for the longitudinally (A) and orthogonally (B) oriented PECUU patches, and for the bi-layered ECM patch (C).

## DETAILED DESCRIPTION

The use of numerical values in the various ranges specified in this application, unless expressly indicated otherwise, are stated as approximations as though the minimum and maximum values within the stated ranges are both preceded by the word "about". In this manner, slight variations above and below the stated ranges can be used to achieve substantially the same results as values within the ranges. Also, unless indicated otherwise, the disclosure of these ranges is intended as a continuous range including every value between the minimum and maximum values. For definitions provided herein, those definitions refer to word forms, cognates and grammatical variants of those words or phrases. As used herein "a" and "an" refer to one or more.

As used herein, the term "patient" or "subject" refers to members of the animal kingdom including but not limited to human beings and "mammal" refers to all mammals, including, but not limited to human beings.

As used herein, the "treatment" or "treating" of a wound or defect means administration to a patient by any suitable dosage regimen, procedure and/or administration route of a composition, device or structure with the object of achieving a desirable clinical/medical end-point, including attracting progenitor cells, healing a wound, correcting a defect, amelioration of one or more sequelae of an injury or defect, *etc.*

As used herein, the terms "comprising," "comprise" or "comprised," and variations thereof, are open ended and do not exclude the presence of other elements not identified. In contrast, the term "consisting of" and variations thereof is intended to be closed, and excludes additional elements in anything but trace amounts. The terms "a" and "an" are intended to refer to one or more.

By "biocompatible", it is meant that a device, scaffold composition, *etc.* is essentially, practically (for its intended use) and/or substantially non-toxic, non-injurious or non-inhibiting or non-inhibitory to cells, tissues, organs, and/or organ systems that would come into contact with the device, scaffold, composition, *etc.,* as described herein.

As used herein, the term "polymer composition" is a composition comprising one or more polymers. As a class, "polymers" includes, without limitation, homopolymers, heteropolymers, co-polymers, block polymers, block co-polymers and can be both natural and synthetic. Homopolymers contain one type of building block, or monomer, whereas co-polymers contain more than one type of monomer. A biological polymer is a naturally-occurring polymer that can be found in a living organism, even though it may be synthesized by man, for example by recombinant means, by *de-novo* synthesis, *etc.* As an example, collagen is a biological polymer, even though it may be manufactured by procession of tissue or by other methods, and includes proteins or peptides having a natural (found in nature) collagen sequence, whether or not co- or post-translationally modified, or in the form of procollagen or mature collagen. A synthetic polymer is a polymer that does not occur in nature.

A polymer "comprises" or is "derived from" a stated monomer if that monomer is incorporated into the polymer. Thus, the incorporated monomer (e.g., "residue") that the polymer comprises is not the same as the monomer prior to incorporation into a polymer, in that at the very least, certain linking groups are introduced and/or are incorporated into the polymer backbone or certain moieties of the monomer are removed in the polymerization process. A polymer is said to comprise a specific type of linkage if that linkage is present in the polymer.

As described herein, a "fiber" is an elongated, slender, thread-like and/or filamentous structure. A "matrix" is any two- or three-dimensional arrangement of elements (e.g., fibers), either ordered (e.g., in a woven or non-woven mesh) or randomly-arranged (as is typical with a mat of fibers typically produced by electrospinning) and can be isotropic or anisotropic.

By "biodegradable or "bioerodable", it is meant that a polymer, once implanted and placed in contact with bodily fluids and tissues in a patient and/or *in vitro,* will degrade either partially or completely through chemical reactions with the bodily fluids and/or tissues, typically and often preferably over a time period of hours, days, weeks or months. Non-limiting examples of such chemical reactions include acid/base reactions, hydrolysis reactions, and enzymatic cleavage. The biodegradation rate of the polymer matrix may be manipulated, optimized or otherwise adjusted so that the matrix degrades over a useful time period. The polymer or polymers typically will be selected so that it degrades in situ over a time period to optimize mechanical conditioning of the tissue and/or growth or repair of tissue. According to a first aspect, the structures described herein comprise a synthetic, biocompatible polymer component and an ECM component. As used herein, structures comprising the described polymeric components and ECM material are generically referred to herein as "scaffolds" and "structures," which includes as a class, without limitation, scaffolds, matrices, biological scaffolds or matrices, cell growth scaffolds or matrices, *etc.* As described herein, according to one aspect, the synthetic, biocompatible polymer is electrospun and the ECM material, *e.g.,* solubilized ECM, such as an ECM gel, is sprayed, e.g. electrosprayed.

Therefore, according to one aspect of the invention, provided herein is a bilayer medical device, i.e. a cardiac patch, for use in treating ischemic cardiomyopathy as well as for treatment of other conditions. The medical device comprises a first, support layer and a second, ECM-containing layer over the first layer. The first layer is a network of synthetic, biocompatible fibers that is anisotropic, and comprises an aqueous solution such as saline (*e.g.,* normal saline). The second layer comprises an ECM material, i.e. an acid protease-solubilized ECM, comprises a network of synthetic, biocompatible fibers that may or may not be the same material as the polymer composition used to produce the network of synthetic, biocompatible fibers of the first layer, and is optionally isotropic. A method of making the device also is provided, as described herein, comprising depositing the described first layer onto a substrate, and subsequently depositing the described second layer over the first layer. The order of deposition can be reversed. Figure 1A is a schematic illustration of the device 10 (not to scale), showing the first layer 20 comprising a network of synthetic, biocompatible fibers, and second layer 30 comprising an ECM material, with the ECM side 35 of the device depicted. Figure 1B is a schematic diagram of the device 10, as in Figure 1A, but placed onto heart 40, (showing only a portion of the heart wall), with the ECM side 35 of the device in contact with (distal to) a surface 45 of the heart 40.

Methods of treatment of ischemic cardiomyopathy also are provided in which the according to any aspect described herein, is placed over an infarct, with the ECM-containing side (*e.g.,* second layer) in contact with (distal to) the heart.

The lack of adequate mechanics limits their therapeutically effects in terms of preventing the progressive LV pathological dilation and increases the risk of mechanical mismatch at the tissue-patch interface. Similarly, non-bioactive materials tend to be encapsulated as a foreign body hindering the potential for tissue functional recovery (e.g. physiological electrical signal propagation and active mechanical component in systole) and increasing the scar tissue volume. In contrast, the various aspects of the invention described herein addresses these issues by (a) offering the capacity to recapitulating native mechanics with the highly organized fibrous electrospun layer and (b) providing an micro - environment conducive for host cell recruitment by the cECM rich layer. An "open-phase" structure (ECM rich layer implanted facing toward the ischemic area and the polymer rich layer providing mechanical support) facilitate the direct interaction between the scaffold and the treated area. Among other benefits, the cardiac patch structure and mechanics described herein supports the following: mitigation of LV wall thinning; mitigation of scar formation and promotion of host cell infiltration; improvement of cardiac function; and prevention of LV dilation. The approach introduces bioengineering strategies for ischemic heart failure which, in the near future, have the potential to be translated to the bedside, reducing patients' post-infarction mortality.

Devices and related methods are provided that are useful for enhancing or maintaining cardiac or cardiovascular function due to disease or injury. The device is a cardiac patch capable of providing mechanical support as well as encouraging tissue growth or cell survival. The patch may also comprise drugs or cells, which also contribute to providing therapeutic or prophylactic treatments.

In one aspect of the device and method, the patch comprises a first polymer layer comprising a network of synthetic, biocompatible fibers such as a poly(ester urethane) urea (PEUU), a poly(ether ester urethane)urea (PEEUU), a poly(ester carbonate)urethane urea (PECUU) and/or a poly(carbonate)urethane urea (PCUU) elastomer fibers. In another aspect, the polymer composition is functionalized with an adhesion-promoting peptide, such as RGD. The composition can be porous, for example and without limitation, the polymer composition may have a porosity of approximately 85%. In one aspect, the first polymer layer comprises fibers, such as electrodeposited fibers, of the synthetic polymer composition. In a further aspect, the fibers are anisotropic. The device and method also comprises a second layer of an extracellular matrix (ECM) material, prepared by digesting (solubilizing) decellularized/devitalized tissue, such as cardiac tissue or LV tissue, using an acid protease such as pepsin, cooling the digested ECM below a gelation temperature of the solubilized ECM once it is neutralized, for instance at room temperature, and neutralizing the ECM. The neutralized ECM can be deposited on the first polymeric layer, *e.g.,* by electrospraying, and placed at a temperature above the gelation temperature (*e.g.,* LCST) of the neutralized ECM.

The patch may be prepared by any useful method, such as by electrospinning/electrospraying. Also provided is a method of making a patch or device as described herein by electrospinning the first polymer layer and electrospraying the acid protease-solubilized ECM onto the electrospun first polymer layer, or vice versa.

Also provided is a method for treating a cardiac or cardiovascular condition. The method comprises implanting a patch at or adjacent to the tissue damage or defect. In one non-limiting embodiment, the damage or defect is in a left ventricular region of a heart which can result from a myocardial infarction. The damage or defect in the cardiac or cardiovascular tissue may be a deficiency resulting from a congenital defect. In one aspect, a method is provided for treating a heart condition, disease or defect in a patient, such as an infarct, comprising attaching the device as described above to the heart of the patient on, about, over or adjacent to a lesion on the heart, such as an infarct, with the second layer of an extracellular matrix (ECM) material contacting the heart with the first polymer layer distal to the heart.

An "ECM material," is a material comprising or that is prepared from an extracellular matrix-containing tissue, and does not consist of a single, isolated and purified ECM component, such as a purified collagen preparation, as are commercially available. Any type of tissue-derived material can be used to produce the ECM materials in the methods, compositions and devices as described herein (*see generally,* US Patent Nos. 4,902,508; 4,956,178; 5,281,422; 5,352,463; 5,372,821; 5,554,389; 5,573,784; 5,645,860; 5,771,969; 5,753,267; 5,762,966; 5,866,414; 6,099,567; 6,485,723; 6,576,265; 6,579,538; 6,696,270; 6,783,776; 6,793,939; 6,849,273; 6,852,339; 6,861,074; 6,887,495; 6,890,562; 6,890,563; 6,890,564; and 6,893,666). In certain embodiments, the ECM material is isolated from a vertebrate animal, for example and without limitation, from a mammal, including, but not limited to, human, monkey, pig, cow and sheep. The ECM material can be prepared from any organ or tissue, including without limitation, urinary bladder, intestine, liver, esophagus and dermis. In one aspect, the ECM material is prepared from heart tissue and therefore is referred to herein as cardiac ECM, and in another aspect, it is left ventricle heart tissue and therefore is referred to herein as left ventricle ECM, for example in either case obtained from a pig, or from any mammal.

In various aspects, ECM material is decellularized, sterilized and/or dried by any useful method. ECM-derived material can then be used in any form in the methods and compositions described herein. In certain aspects, in the context of depositing the ECM material to prepare the device described herein, and to facilitate spraying or electrospraying the ECM material, the ECM material is either finely comminuted, e.g. into micro-scale-sized (from 1-999 microns) or nano-scale-sized (from 1-999 nanometers) particles, or is solubilized, for example in the form of a pre-gel or gel, as described below.

The ECM material can be sterilized by any of a number of standard methods without loss of its ability to induce endogenous tissue growth. For example, the material can be sterilized by propylene oxide or ethylene oxide treatment, gamma irradiation treatment (.05 to 4 mRad), gas plasma sterilization, peracetic acid sterilization, or electron beam treatment. Traditionally, ECM material is disinfected by immersion in 0.1% (v/v) peracetic acid ($\sigma$), 4% (v/v) ethanol, and 96% (v/v) sterile water for 2 h. The ECM material is then washed twice for 15 min with PBS (pH = 7.4) and twice for 15 min with deionized water.

Commercially available ECM preparations can also be used in various aspects of the methods, devices and compositions described herein. In one aspect, the ECM is derived from small intestinal submucosa or SIS. Commercially available preparations include, but are not limited to, Surgisis™, Surgisis-ES™, Stratasis™, and Stratasis-ES™ (Cook Urological Inc.; Indianapolis, Indiana) and GraftPatch™ (Organogenesis Inc.; Canton Massachusetts). In another embodiment, the ECM is derived from dermis. Commercially available preparations include, but are not limited to Pelvicol™ (crosslinked porcine dermal collagen, sold as Permacol™ in Europe; Bard Medical Division, Covington, GA), Repliform™ (Microvasive; Boston, Massachusetts) and Alloderm™ (LifeCell; Branchburg, New Jersey). In another embodiment, the ECM is derived from urinary bladder. Commercially available preparations include, but are not limited to UBM (Acell Corporation; Jessup, Maryland).

The ECM-containing layer is prepared in one aspect from an extracellular matrix-derived gel. In its broadest sense, ECM-derived scaffold materials are comminuted and solubilized to form a hydrogel. The solubilized hydrogel may or may not be dialyzed at any stage prior to solubilization, and in one aspect is not dialyzed prior to or after solubilization. Solubilization may be achieved by digestion with a suitable protease, such as the endoproteases trypsin, chymotrypsin, pepsin, papain and elastase. In certain non-limiting aspects, the method for making such a gel comprises: (i) comminuting devitalized and/or decellularized tissue, (ii) solubilizing non-dialyzed or non-cross-linked devitalized and/or decellularized tissue by digestion with an acid protease in an acidic solution to produce a digest solution, (iii) raising the pH of the digest solution to a pH between 7.2 and 7.8 to produce a neutralized digest solution, and (iv) gelling the solution at a temperature greater than the gelation temperature (*e.g.,* LCST) of the neutralized digest solution, typically greater than 25°C or room temperature, thereby producing an ECM gel.

The devitalized and/or decellularized tissue, e.g., cardiac or left ventricle tissue is solubilized with an acid protease. The acid protease may be, without limitation, pepsin or trypsin, and in one embodiment is pepsin. The material typically is solubilized at an acid pH suitable or optimal for the protease, such as greater than about pH 2, or between pH 2 and 4, for example in a 0.01M HCl solution. The material typically is solubilized for 12-48 hours, depending upon the tissue type (e.g., see examples below), with mixing (stirring, agitation, admixing, blending, rotating, tilting, etc.) often on ice, at 4°C or at room temperature (e.g., 20°C to 23°C). Once the material is solubilized, the pH is raised to between 7.2 and 7.8, and according to one embodiment, to pH 7.4. Bases, such as bases containing hydroxyl ions, including NaOH, can be used to raise the pH of the solution. Likewise buffers, such as an isotonic buffer, including, without limitation, Phosphate Buffered Saline (PBS), can be used to bring the solution to a target pH, or to aid in maintaining the pH and ionic strength of the gel to target levels, such as physiological pH and ionic conditions. The neutralized digest solution can be gelled at temperatures approaching 37°C, typically at any temperature over 25°C, though gelation proceeds much more rapidly at temperatures over 30°C, and as the temperature approaches physiological temperature. The method, according to one aspect, does not include a dialysis step prior to gelation, yielding a more-complete ECM-like matrix that typically gels at 37°C more slowly than comparable collagen or dialyzed ECM preparations.

The ECM gel can be sprayed as a liquid or hydrogel in the methods provided herein. An ECM gel may have an LCST (Lower Critical Solution Temperature) above or below the temperature at which the solution is sprayed, and as such will have a gel transition at a temperature higher, equal to or lower than the temperature at which the ECM gel is sprayed. For example, if the hydrogel is sprayed at room temperature (that is approximately 20-25°C) or less and the LCST of the ECM material is greater than the spraying temperature, but, e.g., less than 37°C, the material can be sprayed and will later gel on warming, for example on implantation or when placed in an warm environment, e.g. at 37° in an incubator. Thus, an ECM gel with an LCST between 20°C and 37°C, for example and without limitation approximately 25°C, is useful in the compositions and methods described herein. See, e.g. United States Patent Publication No. 2008260831. See also, Stankus et al., Hybrid nanofibrous scaffolds from electrospinning of a synthetic biodegradable elastomer and urinary bladder matrix, J Biomater. Sci. Polym. Ed. (2008) 19(5):635-652.

Any biocompatible polymer composition can be used to produce the synthetic, biocompatible fibers described herein. Non-limiting examples of useful polymer compositions for use in the synthetic, biocompatible fibers include: polyolefin (polyalkene), polyester, polycarbonate, polyanhydride, polyether, polyurea, polyurethane, polyketone, and fluoropolymers. In one aspect, the polymer composition is bioerodible. Non-limiting examples of biocompatible, bioerodible , elastomeric (co)polymer compositions that have been established as useful in preparing cell growth matrices and therefore are useful in the first and second polymeric layers, include poly(ester urethane) urea (PEUU), poly(ether ester urethane)urea (PEEUU), poly(ester carbonate)urethane urea (PECUU) and poly(carbonate)urethane urea (PCUU). In general, useful (co)polymers including, without limitation: polymers comprising monomers of alpha-hydroxy acids; polylactides, such as poly(lactide-co-glycolide), poly(L-lactide-co-caprolactone), polyglycolic acid, poly(dl-lactide-co-glycolide), poly(l-lactide-co-dl-lactide); polyesters including polyhydroxybutyrate, polyhydroxyvalerate, polydioxanone and polyglactin; polylactones including polycaprolactone; polycarbonates, polyglyconate, poly(glycolide-co-trimethylene carbonate), poly(glycolide-co-trimethylene carbonate-co-dioxanone); and polyurethanes, poly(ester urethane) urea elastomer.

In certain aspects, the polymers used to make the structures described herein also release therapeutic agents when they degrade within the patient's body. For example, the individual building blocks of the polymers may be chosen such that the building blocks themselves provide a therapeutic benefit when released in situ through the degradation process. In one embodiment, one of the polymer building blocks is putrescine, which has been implicated as a substance that causes cell growth and cell differentiation.

In one aspect, the fibers of the network of synthetic, biocompatible fibers of the first and/or second layer comprise a biodegradable poly(ester urethane) urea elastomer (PEUU). PEUU can be manufactured by reacting a diol with a diisocyanate to form a prepolymer and then reacting the prepolymer with a diamine. A non-limiting example of such a PEUU is an elastomeric polymer made from polycaprolactone diol (MW 2000) and 1,4-diisocyanatobutane, using a diamine chain extender such as putrescine. One non-limiting example or a method for preparing a PEUU polymer is a two-step polymerization process whereby polycaprolactone diol (MW 2000), 1,4-diisocyanatobutane, and diamine are combined in a 2:1:1 molar ratio. In the first step to form the prepolymer, a 15 wt% solution of 1,4-diisocyanatobutane in DMSO (dimethyl sulfoxide) is stirred continuously with a 25 wt% solution of polycaprolactone diol in DMSO. Then, stannous octoate is added and the mixture is allowed to react at 75°C for 3 hours. In the second step, the prepolymer is reacted with a diamine to extend the chain and to form the polymer. In one embodiment, the diamine is putrescine, which is added drop-wise while stirring and allowed to react at room temperature for 18 hours. In one aspect, the diamine is lysine ethyl ester, which is dissolved in DMSO with triethylamine, added to the prepolymer solution, and allowed to react at 75°C for 18 hours. After the two step polymerization process, the polymer solution is precipitated in distilled water. Then, the wet polymer is immersed in isopropanol for three days to remove any unreacted monomers. Finally, the polymer is dried under vacuum at 50°C for 24 hours.

In another aspect, the fibers of the network of synthetic, biocompatible fibers of the first and/or second layer comprise a poly(ether ester urethane) urea elastomer (PEEUU). For example and without limitation, the PEEUU may be made by reacting polycaprolactone-b-polyethylene glycol-b-polycaprolactone triblock copolymers with 1,4-diisocyanatobutane and putrescine. In one non-limiting embodiment, PEEUU is obtained by a two-step reaction using a 2:1:1 reactant stoichiometry of 1,4-diisocyanatobutane:triblock copolymer:putrescine. According to one non-limiting embodiment, the triblock polymer can be prepared by reacting poly(ethylene glycol) and $\varepsilon$-caprolactone with stannous octoate at 120°C for 24 hours under a nitrogen environment. The triblock copolymer is then washed with ethyl ether and hexane, then dried in a vacuum oven at 50°C. In the first step to form the prepolymer, a 15 wt% solution of 1,4-diisocyanatobutane in DMSO is stirred continuously with a 25 wt% solution of triblock copolymer in DMSO. Then, stannous octoate is added and the mixture is allowed to react at 75°C for 3 hours. In the second step, putrescine is added drop-wise under stirring to the prepolymer solution and allowed to react at room temperature for 18 hours. The PEEUU polymer solution is then precipitated with distilled water. The wet polymer is immersed in isopropanol for 3 days to remove unreacted monomer and dried under vacuum at 50°C for 24 hours.

In another aspect, the fibers of the network of synthetic, biocompatible fibers of the first and/or second layer comprise a poly(ester carbonate)urethane urea (PECUU) or a poly(carbonate)urethane urea (PCUU), which are described, for example, in Hong et al. (Tailoring the degradation kinetics of poly(ester carbonate urethane)urea thermoplastic elastomers for tissue engineering scaffolds Biomaterials, Biomaterials 31 (2010) 4249-4258). Poly(ester carbonate urethane)urea (PECUU) is synthesized, for example using a blended soft segment of polycaprolactone (PCL) and poly(1,6-hexamethylene carbonate) (PHC) and a hard segment of 1,4-diisocyanatobutane (BDI) with chain extension by putrescine. Different molar ratios of PCL and PHC can be used to achieve different physical characteristics. Putrescine is used as a chain extender by a two-step solvent synthesis method. In one example, the (PCL + PHC):BDI:putrescine molar ratio is defined as 1:2:1. Variable molar ratios of PCL and PHC (e.g., PCL/PHC ratios of 100/0 (yielding a PEUU), 75/25, 50/50, 25/75 and 0/100 (yielding a PCUU)) are completely dissolved in DMSO in a 3-neck flask with argon protection and then BDI is added to the solution, following 4 drops of $Sn(Oct)_2$. The flask is placed in an oil bath at 70°C. After 3 h, the prepolymer solution is cooled at room temperature and then a putrescine/DMSO solution is added dropwise into the agitated solution. The final polymer solution concentration is controlled to be approximately 4% (w/v). The flask is then placed in an oil bath and kept at 70°C overnight. The polymer is precipitated in an excess volume of cool deionized water and then dried in a vacuum at 60°C for 3 days. The polyurethane ureas synthesized from the different PCL/PHC molar ratios defined above are referred to as PEUU, PECUU 75/25, PECUU 50/50, PECUU 25/75 and PCUU, respectively. In practice, the yields of all final products using this method is approximately 95%.

Diamines and diols also are useful building blocks for preparing the fibers of the network of synthetic, biocompatible fibers of the first and/or second layers. Diamines as described above have the structure $H_2N$-R-$NH_2$ where "R" is an aliphatic or aromatic hydrocarbon or a hydrocarbon comprising aromatic and aliphatic regions. The hydrocarbon may be linear or branched. Examples of useful diamines are putrescine (R=butylene) and cadaverine (R=pentylene). Useful diols include polycaprolactone (e.g., Mw 1000-5000), multi-block copolymers, such as polycaprolactone-PEG copolymers, including polycaprolactone-b-polyethylene glycol-b-polycaprolactone triblock copolymers of varying sizes. Other building blocks for useful diols include, without limitation glycolides (e.g. polyglycolic acid (PGA)), lactides, dioxanones, and trimethylene carbonates. Diisocyanates have the general structure OCN-R-NCO, where "R" is an aliphatic or aromatic hydrocarbon or a hydrocarbon comprising aromatic and aliphatic regions. The hydrocarbon may be linear or branched.

In additional aspects, the fibers of the network of synthetic, biocompatible fibers of the first layer comprise of the first and/or second layer comprise polyethylene terephthalate (PET, e.g., DACRON). Of note, PET is less biodegradable than the copolymers described above, and is stiffer. PET scaffolds structures are made essentially in the manner described herein for PEUU and other polymer compositions described herein. Polymer concentrations and infusion rates may be altered to accommodate the different qualities of the PET composition, for example and without limitation, for PET, 20% w/v in HFIP at 12 mL/h infusion rate, as used in the examples below.

In other aspects, the fibers of the network of synthetic, biocompatible fibers of the first and/or second layer comprise a tyrosine polyarylate (TPA). As with PET, TPA is less biodegradable than the polyurethane copolymers described above, and also is stiffer. TPA scaffolds structures are made essentially in the manned described herein for PEUU and other polymer compositions. Polymer concentrations and infusion rates may be altered to accommodate the different qualities of the TPA composition, for example and without limitation, for TPA, 12% w/v in HFIP at 20 mL/h infusion rate, as used in the examples below. Tyrosine polyarylates are commonly prepared from an aliphatic acid and a tyrosine-derived diphenol. Non-limiting examples of useful aliphatic acids include: succinic acid, adipic acid, sebacic acid, and dicarboxylic acid chlorides or anhydrides. Non-limiting examples of tyrosine-derived diphenols include desaminotyrosyl-tyrosine alkyl esters, where the alkyl is, for example, one of ethyl, hexyl and octyl (DTE). As an example, in the Examples below, Poly(DTE-co-27.5 DT succinate) was used. TPAs and methods of making TPAs are described, for example, in United States Patent No. 5,216,115 and United States Patent Publication No. 20110082545 disclose useful TPAs. Additional references disclosing TPA compositions and methods of making and using those compositions include: Fiordeliso, J, et al. (1994) Design, synthesis, and preliminary characterization of tyrosine-containing polyarylates: new biomaterials for medical applications, J Biomater Sci Polym Ed. 1994;5(6):497-510; Huang, X et al. (2009) A library of L-tyrosine-derived biodegradable polyarylates for potential biomaterial applications, part I: synthesis, characterization and accelerated hydrolytic degradation J Biomater Sci Polym Ed. 2009;20(7-8):935-55; and Bourke, SL et al. (2003) Polymers derived from the amino acid L-tyrosine: polycarbonates, polyarylates and copolymers with poly(ethylene glycol) Adv Drug Deliv Rev. 2003 Apr 25;55(4):447-66.

In further aspects, the fibers of the network of synthetic, biocompatible fibers of the first and/or second layer comprise a combination of two or more polymer compositions as described above, for example combinations of two or more different PEUU, PEEUU, PECUU or PCUU copolymer compositions (e.g., two different PECUU copolymer compositions, or a PECUU copolymer combined with a PCUU copolymer), or a PEUU, PEEUU, PECUU or PCUU composition combined with one or both of a PET or TPA copolymer composition. The network of synthetic, biocompatible fibers can comprise multiple layers of different composition, orientation, porosity, fiber thickness, etc.

In another aspect, at least one therapeutic agent is added to the scaffold or composition described herein, such as admixed with the network of synthetic, biocompatible fibers of the first and/or second layer and/or the ECM layer, before it is implanted in the patient or otherwise administered to the patient. Generally, the therapeutic agents include any substance that can be coated on, embedded into, absorbed into, adsorbed to, or otherwise attached to or incorporated onto or into the structure or incorporated into a drug product that would provide a therapeutic benefit to a patient. Non-limiting examples of such therapeutic agents include antimicrobial agents, growth factors, emollients, retinoids, and topical steroids. Each therapeutic agent may be used alone or in combination with other therapeutic agents. Alternatively, the therapeutic agent may be blended with the polymer while a polymer is being processed. For example, the therapeutic agent may be dissolved in a solvent (e.g., DMSO) and added to the polymer blend during processing. In another embodiment, the therapeutic agent is mixed with a carrier polymer (e.g., polylactic-glycolic acid microparticles) which is subsequently processed with a synthetic, biocompatible fiber, as described herein r. By blending the therapeutic agent with a carrier polymer or the synthetic, biocompatible fiber, as described herein, itself, the rate of release of the therapeutic agent may be controlled by the rate of polymer degradation.

Active agents that may be incorporated into the compositions described herein include, without limitation, anti-inflammatories, such as, without limitation, nitro-fatty acids NSAIDs (non-steroidal anti-inflammatory drugs) such as salicylic acid, indomethacin, sodium indomethacin trihydrate, salicylamide, naproxen, colchicine, fenoprofen, sulindac, diflunisal, diclofenac, indoprofen sodium salicylamide, antiinflammatory cytokines, and antiinflammatory proteins or steroidal anti-inflammatory agents); antibiotics; anticlotting factors such as heparin, Pebac, enoxaprin, aspirin, hirudin, plavix, bivalirudin, prasugrel, idraparinux, warfarin, coumadin, clopidogrel, PPACK, GGACK, tissue plasminogen activator, urokinase, and streptokinase; growth factors. Other active agents include, without limitation: (1) immunosuppressants; glucocorticoids such as hydrocortisone, betamethisone, dexamethasone, flumethasone, isoflupredone, methylpred-nisolone, prednisone, prednisolone, and triamcinolone acetonide; (2) antiangiogenics such as fluorouracil, paclitaxel, doxorubicin, cisplatin, methotrexate, cyclophosphamide, etoposide, pegaptanib, lucentis, tryptophanyl-tRNA synthetase, retaane, CA4P, AdPEDF, VEGF-TRAP-EYE, AG-103958, Avastin, JSM6427, TG100801, ATG3, OT-551, endostatin, thalidomide, becacizumab, neovastat; (3) antiproliferatives such as sirolimus, paclitaxel, perillyl alcohol, farnesyl transferase inhibitors, FPTIII, L744, antiproliferative factor, Van 10/4, doxorubicin, 5-FU, Daunomycin, Mitomycin, dexamethasone, azathioprine, chlorambucil, cyclophosphamide, methotrexate, mofetil, vasoactive intestinal polypeptide, and PACAP; (4) antibodies; (5) drugs acting on immunophilins, such as cyclosporine, zotarolimus, everolimus, tacrolimus and sirolimus (rapamycin), interferons, TNF binding proteins; (6) taxanes, such as paclitaxel and docetaxel; statins, such as atorvastatin, lovastatin, simvastatin, pravastatin, fluvastatin and rosuvastatin; (7) nitric oxide donors or precursors, such as, without limitation, Angeli's Salt, L-Arginine, Free Base, Diethylamine NONOate, Diethylamine NONOate/AM, Glyco-SNAP-1, Glyco-SNAP-2, S-Nitroso-N-acetylpenicillamine, S-Nitrosoglutathione, NOC-5, NOC-7, NOC-9, NOC-12, NOC-18, NOR-1, NOR-3, SIN-1, Hydrochloride, Sodium Nitroprusside, Dihydrate, Spermine NONOate, Streptozotocin; and (8) antibiotics, such as, without limitation: acyclovir, afloxacin, ampicillin, amphotericin B, atovaquone, azithromycin, ciprofloxacin, clarithromycin, clindamycin, clofazimine, dapsone, diclazaril, doxycycline, erythromycin, ethambutol, fluconazole, fluoroquinolones, foscarnet, ganciclovir, gentamicin, iatroconazole, isoniazid, ketoconazole, levofloxacin, lincomycin, miconazole, neomycin, norfloxacin, ofloxacin, paromomycin, penicillin, pentamidine, polymixin B, pyrazinamide, pyrimethamine, rifabutin, rifampin, sparfloxacin, streptomycin, sulfadiazine, tetracycline, tobramycin, trifluorouridine, trimethoprim sulfate, Zn-pyrithione, ciprofloxacin, norfloxacin, afloxacin, levofloxacin, gentamicin, tobramycin, neomycin, erythromycin, trimethoprim sulfate, polymixin B and silver salts such as chloride, bromide, iodide and periodate.

Any useful cytokine or chemoattractant can be mixed into, mixed with, or otherwise combined with any composition as described herein. For example and without limitation, useful components include growth factors, interferons, interleukins, chemokines, monokines, hormones, and angiogenic factors. In certain non-limiting aspects, the therapeutic agent is a growth factor, such as a neurotrophic or angiogenic factor, which optionally may be prepared using recombinant techniques. Non-limiting examples of growth factors include basic fibroblast growth factor (bFGF), acidic fibroblast growth factor (aFGF), vascular endothelial growth factor (VEGF), hepatocyte growth factor (HGF), insulin-like growth factors 1 and 2 (IGF-1 and IGF-2), platelet derived growth factor (PDGF), stromal derived factor 1 alpha (SDF-1 alpha), nerve growth factor (NGF), ciliary neurotrophic factor (CNTF), neurotrophin-3, neurotrophin-4, neurotrophin-5, pleiotrophin protein (neurite growth-promoting factor 1), midkine protein (neurite growth-promoting factor 2), brain-derived neurotrophic factor (BDNF), tumor angiogenesis factor (TAF), corticotrophin releasing factor (CRF), transforming growth factors $\alpha$ and $\beta$ (TGF-$\alpha$ and TGF-$\beta$), interleukin-8 (IL-8), granulocyte-macrophage colony stimulating factor (GM-CSF), interleukins, and interferons. Commercial preparations of various growth factors, including neurotrophic and angiogenic factors, are available from R & D Systems, Minneapolis, Minnesota; Biovision, Inc, Mountain View, California; ProSpec-Tany TechnoGene Ltd., Rehovot, Israel; and Cell Sciences®, Canton, Massachusetts.

In certain non-limiting aspects, the therapeutic agent is an antimicrobial agent, such as, without limitation, isoniazid, ethambutol, pyrazinamide, streptomycin, clofazimine, rifabutin, fluoroquinolones, ofloxacin, sparfloxacin, rifampin, azithromycin, clarithromycin, dapsone, tetracycline, erythromycin, ciprofloxacin, doxycycline, ampicillin, amphotericin B, ketoconazole, fluconazole, pyrimethamine, sulfadiazine, clindamycin, lincomycin, pentamidine, atovaquone, paromomycin, diclazaril, acyclovir, trifluorouridine, foscarnet, penicillin, gentamicin, ganciclovir, iatroconazole, miconazole, Zn-pyrithione, and silver salts such as chloride, bromide, iodide and periodate.

In certain non-limiting aspects, the therapeutic agent is an anti-inflammatory agent, such as, without limitation, an NSAID, such as salicylic acid, indomethacin, sodium indomethacin trihydrate, salicylamide, naproxen, colchicine, fenoprofen, sulindac, diflunisal, diclofenac, indoprofen, sodium salicylamide; an anti-inflammatory cytokine; an anti-inflammatory protein; a steroidal anti-inflammatory agent; or an anti-clotting agents, such as heparin. Other drugs that may promote wound healing and/or tissue regeneration may also be included.

Non-limiting examples of antiangiogenic agents include: Macugen (pegaptanib sodium); Lucentis; Tryptophanyl-tRNA synthetase (TrpRS); AdPEDF; VEGF TRAP-EYE; AG-013958; Avastin (bevacizumab); JSM6427; TG100801; ATG3; Perceiva (originally sirolimus or rapamycin); E10030, ARC1905 and colociximab (Ophthotech) and Endostatin. Ranibizumab is currently the standard in the United States for treatment of neovascular AMD. It binds and inhibits all isoforms of VEGF. Although effective in many cases, treatment with ranibizumab requires sustained treatment regimens and frequent intravitreal injections. VEGF Trap is a receptor decoy that targets VEGF with higher affinity than ranibizumab and other currently available anti-VEGF agents. Blocking of VEGF effects by inhibition of the tyrosine kinase cascade downstream from the VEGF receptor also shows promise, and includes such therapies as vatalanib, TG100801, pazopanib, AG013958 and AL39324. Small interfering RNA technology-based therapies have been designed to downregulate the production of VEGF (bevasiranib) or VEGF receptors (AGN211745). Other potential therapies include pigment epithelium-derived factor-based therapies, nicotinic acetylcholine receptor antagonists, integrin antagonists and sirolimus. (See, e.g., Chappelow, AV, et al. Neovascular age-related macular degeneration: potential therapies, Drugs. 2008;68(8):1029-36 and Barakat MR, et al. VEGF inhibitors for the treatment of neovascular age-related macular degeneration, Expert Opin Investig Drugs. 2009 May;18(5):637-46.

In another aspect, antioxidants are added to the polymeric composition, such as organic or inorganic antioxidants. In one aspect, the antioxidant is a nanoparticle incorporated by any means into the polymer composition, such as, for example, a cerium nanoparticle. As an example, an anisotropic heart valve or heart valve leaflet prosthesis is manufactured by electro spinning, or by any useful method, and cerium nanoparticles are deposited in and/or on the prosthesis either during or after manufacture.

Pharmaceutically acceptable salts of any active agent (e.g., therapeutic agent or drug), bound to or otherwise combined with, or incorporated into the composition according to any aspect herein, may be employed. Pharmaceutically acceptable salts are, because their solubility in water is greater than that of the initial or basic compounds, particularly suitable for medical applications. These salts have a pharmaceutically acceptable anion or cation. Suitable pharmaceutically acceptable acid addition salts of the compounds of the invention include, without limitation, salts of inorganic acids such as hydrochloric acid, hydrobromic, phosphoric, metaphosphoric, nitric and sulfuric acid, and of organic acids such as, for example, acetic acid, benzenesulfonic, benzoic, citric, ethanesulfonic, fumaric, gluconic, glycolic, isethionic, lactic, lactobionic, maleic, malic, methanesulfonic, succinic, p-toluenesulfonic and tartaric acid. Suitable pharmaceutically acceptable basic salts include without limitation, ammonium salts, alkali metal salts (such as sodium and potassium salts), alkaline earth metal salts (such as magnesium and calcium salts), and salts of trometamol (2-amino-2-hydroxymethyl-1,3-propanediol), diethanolamine, lysine or ethylenediamine. Pharmaceutically acceptable salts may be prepared from parent compounds by any useful method, as are well known in the chemistry and pharmaceutical arts.

An "active agent" ("drug") is a pharmacologically-active constituent of a drug product (dosage form), and includes any compositions having a preventative or therapeutic effect, including and without limitation, antibiotics, peptides, hormones, organic molecules, vitamins, supplements, factors, proteins and chemoattractants.

The deposited synthetic, biocompatible polymer composition, as described herein, typically is porous. As used herein, the term "porosity" refers to a ratio between a volume of all the pores within the polymer composition and a volume of the whole polymer composition. For instance, a polymer composition with a porosity of 85% would have 85% of its volume containing pores and 15% of its volume containing the polymer. In certain non-limiting embodiments, the porosity of the structure is at least 60%, 65%, 70%, 75%, 80%, 85%, or 90%, or increments therebetween. The structures described herein are manufactured in one aspect by electrospinning. It therefore is often advantageous to adjust the pore size or degree of porosity by varying the polymer concentration of the electrospinning solution or by varying the spinning distance from the nozzle to the target. For example and without limitation, the average pore size may be increased by increasing the amount of polymeric components within the suspension used for electrospinning, which results in larger fiber diameters and therefore larger pore sizes. In another non-limiting example, the average pore size can be increased by increasing spinning distance from the nozzle to the target, which results in less adherence between fibers and therefore a looser matrix. Where ECM material, *e.g.* ECM gel, is co-deposited during the electrospinning, many of the pores (that is a large percentage of the pores or interstices) in the deposited polymer are filled with the ECM gel. It should be noted that although it is stated that the ECM gel is electrosprayed, sprayed, or otherwise deposited, the ECM gel is typically deposited prior to gelation, and is therefore deposited as a pre-gel.

In its simplest sense, electrospinning is caused by the deposit of a liquid composition, such as polymer fibers onto a target surface caused by an electric potential. Electrospinning methods are well-known in the field of tissue engineering and are conducted essentially as described below. Electrospinning permits fabrication of structures that resemble the scale and fibrous nature of native extracellular matrix (ECM). The ECM is composed of fibers, pores, and other surface features at the sub-micron and nanometer size scale. Such features directly impact cellular interactions with synthetic materials such as migration and orientation. Electrospinning also permits fabrication of oriented fibers to result in structures with inherent anisotropy. These aligned structures can influence cellular growth, morphology and ECM production. For example, Xu. *et al.* found smooth muscle cell (SMC) alignment with poly(L-lactide-*co*-ε-caprolactone) fibers (Xu C.Y., Inai R., Kotaki M., Ramakrishna S., "Aligned biodegradable nanofibrous structure: a potential for blood vessel engineering", Biomaterials 2004 (25) 877-86) and Lee *et al.* submitted aligned non-biodegradable polyurethane to mechanical stimulation and found cells cultured on aligned scaffolds produced more ECM than those on randomly organized scaffolds (Lee C.H., Shin H.J., Cho I.H., Kang Y.M. Kim I.A., Park K.D., Shin, J.W., "Nanofiber alignment and direction of mechanical strain affect the ECM production of human ACL fibroblast", Biomaterials 2005 (26) 1261-1270).

The process of electrospinning involves placing a polymer-containing fluid (for example, a polymer solution, a polymer suspension, or a polymer melt) in a reservoir equipped with a small orifice, such as a needle or pipette tip, and a metering pump. One electrode of a high voltage source is also placed in electrical contact with the polymer-containing fluid or orifice, while the opposite electrode is placed in electrical contact with a target (typically a collector screen or rotating mandrel). During electrospinning, the polymer-containing fluid is charged by the application of high voltage to the solution or orifice (for example, about 3-15 kV) and then forced through the small orifice by the metering pump, which provides steady flow. While the polymer-containing fluid at the orifice normally would have a hemispherical shape due to surface tension, the application of the high voltage causes the otherwise hemispherically shaped polymer-containing fluid at the orifice to elongate to form a conical shape known as a Taylor cone. With sufficiently high voltage applied to the polymer-containing fluid and/or orifice, the repulsive electrostatic force of the charged polymer-containing fluid overcomes the surface tension and a charged jet of fluid is ejected from the tip of the Taylor cone and accelerated towards the target, which typically is biased between -2 to -10 kV. Optionally, a focusing ring with an applied bias (for example, 1-10 kV) can be used to direct the trajectory of the charged jet of polymer-containing fluid. As the charged jet of fluid travels towards the biased target, it undergoes a complicated whipping and bending motion. If the fluid is a polymer solution or suspension, the solvent typically evaporates during mid-flight, leaving behind a polymer fiber on the biased target. If the fluid is a polymer melt, the molten polymer cools and solidifies in mid-flight and is collected as a polymer fiber on the biased target. As the polymer fibers accumulate on the biased target, a non-woven, porous mesh is formed on the biased target. Under certain conditions, for instance with solutions lacking sufficient viscosity and/or electrospun with certain tolerances, a fiber is not formed, but a spray is formed, depositing discrete droplets onto the target instead of a fiber. This is electrospraying, which is used in the context of various aspects as described herein to deposit the described ECM material and/or, aqueous solutions, such as saline, cell culture media, or PBS, to be dispersed through the network of synthetic fibers of the first layer as described herein.

The properties of the electrospun structures, e.g., elastomeric scaffolds, can be tailored by varying the electrospinning conditions. For example, when the biased target is relatively close to the orifice, the resulting electrospun mesh tends to contain unevenly thick fibers, such that some areas of the fiber have a "bead-like" appearance. However, as the biased target is moved further away from the orifice, the fibers of the non-woven mesh tend to be more uniform in thickness. Moreover, the biased target can be moved relative to the orifice. In certain non-limiting embodiments, the biased target is moved back and forth in a regular, periodic fashion, such that fibers of the non-woven mesh are substantially parallel to each other. When this is the case, the resulting non-woven mesh may have a higher resistance to strain in the direction parallel to the fibers, compared to the direction perpendicular to the fibers, such that the mesh is anisotropic. In other non-limiting embodiments, the biased target is moved randomly relative to the orifice, so that the resistance to strain in the plane of the non-woven mesh is isotropic. The target can also be electrospun on a rotating mandrel. In this case, the properties of the non-woven mesh may be changed by varying the speed of rotation. The properties of the electrospun structure may also be varied by changing the magnitude of the voltages applied to the electrospinning system. In one non-limiting embodiment, the electrospinning apparatus includes an orifice biased to 12 kV, a target biased to -7 kV, and a focusing ring biased to 3 kV. Moreover, a useful orifice diameter is 0.119 cm (0.047") (I.D.) and a useful target distance is about 10-25 cm. Other electrospinning conditions that can be varied include, for example and without limitation, the feed rate of the polymer solutions, the solution concentrations, and the polymer molecular weight.

Electrospinning can be performed using two or more nozzles, wherein each nozzle is a source of a different polymer solution. The nozzles may be biased with different biases or the same bias in order to tailor the physical and chemical properties of the resulting non-woven polymeric mesh. Additionally, many different targets may be used. In addition to a flat, plate-like target, use of a mandrel or a revolving disk as a target is contemplated.

When the electrospinning is to be performed using a polymer suspension, the concentration of the polymeric component in the suspension can also be varied to modify the physical properties of the scaffold. For example, when the polymeric component is present at relatively low concentration, the resulting fibers of the electrospun non-woven mesh have a smaller diameter than when the polymeric component is present at relatively high concentration. Without wishing to be limited by theory, it is believed that lower concentration solutions have a lower viscosity, leading to faster flow through the orifice to produce thinner fibers. One skilled in the art can adjust polymer concentrations to obtain fibers of desired characteristics. Useful ranges of concentrations for the polymer component are from 1 wt% to 25 wt%, 4 wt% to 20 wt%, and from 10 wt% to 15 wt%, including increments therebetween for all ranges.

As shown below, the structure is produced by electrospinning a polymer suspension comprising a synthetic polymeric component, followed by electrospraying the ECM gel and/or other liquid. The order may be reversed. Non-limiting examples of useful range of high-voltage to be applied to the polymer suspension is from 0.5 to 30 kV, from 5 to 25 kV, and from 10 to 15 kV.

In one aspect, the structure is electrodeposited on a rotating mandrel to produce a tubular structure. The tubular structure is cut to remove it from the mandrel, and circular patches are produced by punching using surgical punchers.

In the electrodeposition process, a multi-layer structure is produced, for example, comprising a first polymeric layer of a network of synthetic, biocompatible fibers, such as a PEUU, PEEUU, PECUU and/or PCUU composition, and a second layer comprising an ECM gel, e.g. a cECM gel. In one aspect, the second layer comprises the ECM material dispersed within an optionally isotropic network of synthetic, biocompatible fibers. The multi-layered structure combines a synthetic, biodegradable polymer composition and an ECM gel. The device described herein may be sterilized by any useful means, and then packaged and distributed, e.g., according to standard usage in the biomedical industry.

In use, in various aspects of the invention, the structures described herein can be used for cell growth and implantation for tissue generation or regeneration. For example, the device can be sutured, glued or otherwise affixed into place at a site of an injury or defect, such as over an infarct, that is on a wall of a heart covering a location in the wall of the heart containing the infarct. In one aspect, the device is placed with the ECM material-containing layer, essentially as depicted in Figure 1B, in contact with the surface of the heart.

Because the structure, in certain aspects, is biodegradable, it is gradually replaced by native-origin tissue and/or supports growth and/or repair of adjacent tissue. If formed into a tubular structure, it may be used to replace blood vessels or portions of a patient's digestive tract and implanted, e.g., by anastomosis. It can also be used as a reinforcement for tissue already present in the patient. The device/scaffolds also may be used *in vitro,* in any suitable cell growth vessel, bioreactor, plate, flask, etc.

One substantial benefit of the device as described herein is that it is effective without pre-seeding with cells and incubating to grow out the cells, but can be implanted cell-free. As such, in one aspect, the device is cell-free, and is implanted cell-free. In one aspect, although not necessary to produce effective results, prior to implantation, the device is placed *ex vivo* in a cell culture vessel and is incubated with media and cells of a patient, and the cells are allowed to grow on, and infiltrate within the scaffold prior to implantation. In another embodiment, the scaffold is placed within a patient and after a desired period of time for cell infiltration, explanted and re-implanted at a different location. In another aspect, the cells are dissolved into an appropriate solution *(e.g.,* a growth medium, buffer, or even the ECM material that is electrosprayed during formation of the scaffold), and then sprayed onto a synthetic, biocompatible polymer scaffold while the scaffold is being formed by electrospinning. This method is particularly suitable when a highly cellularized tissue engineered construct is desired. While pressure spraying (that is, spraying cells from a nozzle under pressure) is contemplated herein, in certain non-limiting embodiments, the cells can be electrosprayed onto the non-woven mesh during electrospinning. As described herein, electrospraying involves subjecting a cell-containing solution with an appropriate viscosity and concentration to an electric field sufficient to produce a spray of small charged droplets of solution that contain cells. Nevertheless, as indicated above, addition of cells prior to implantation is not necessary for effective results.

**Example**

As an intervention to abrogate ischemic cardiomyopathy, the concept of applying a temporary, local patch to the surface of the recently infarcted ventricle has been explored from a number of design perspectives. Two features considered for such a cardiac patch include the provision of appropriate mechanical support and the capacity to influence the remodeling pathway by providing cellular or biomolecule delivery. The following focuses on these two features by first evaluating the incorporation of a cardiac extracellular matrix (ECM) component, and second by evaluating the impact of patch anisotropy on the pathological remodeling process initiated by myocardial infarction. The functional outcomes of microfibrous, elastomeric, biodegradable cardiac patches have been evaluated in a rat chronic infarction model. Ten weeks after infarction and 8 weeks after patch epicardial placement, echocardiographic function, tissue-level structural remodeling (e.g., biaxial mechanical response and microstructural analysis), and cellular level remodeling were assessed. The results showed that the incorporation of a cardiac ECM altered the progression of several keys aspects of maladaptive remodeling following myocardial infarction. This included decreasing LV global mechanical compliance, inhibiting echocardiographically-measured functional deterioration, mitigating scar formation and LV wall thinning, and promoting angiogenesis. In evaluating the impact of patch anisotropy, no effects from the altered patch mechanics were detected after 8 weeks, possibly due to patch fibrous encapsulation. Overall, this study demonstrates the benefit of a cardiac patch design that combines both ventricle mechanical support, through a biodegradable, fibrillary elastomeric component, and the incorporation of ECM-based hydrogel components.

*Materials and Methods:*

1. Polymer synthesis and cardiac ECM extraction

PECUU was synthesized from PCL ($M_n$ = 2000 Sigma), PHC ($M_n$ = 2000 Sigma) and BDI (Sigma) using putrescine as a chain extender as previously described in (Hong Y, et al., Tailoring the degradation kinetics of poly(ester carbonate urethane)urea thermoplastic elastomers for tissue engineering scaffolds. Biomaterials. 2010;31:4249-58). The (PCL+PHC):BDI:putrescine molar ratio was 1:2:1 while the PCL/PHC molar ratio was 50/50.

Intact porcine hearts were obtained from market weight pigs for whole organ decellularization via retrograde aortic perfusion. The aorta was cannulated approximately 2-3 cm above the aortic valve, and the entire heart placed in a 4L beaker of Type I water. All subsequent steps were performed using a peristaltic pump (Masterflex® L/S digital pump system) to perfuse decellularization chemicals through the heart using recirculating flow from the beaker unless otherwise indicated. Solution changes and flow rates during decellularization were applied according to an optimized protocol (Remlinger NT, Wearden PD, Gilbert TW. Procedure for decellularization of porcine heart by retrograde coronary perfusion. J Vis Exp2012. p. e50059) with modification as shown in the Table 1 below.

Table 1. Cardiac perfusion decellularization steps

| Solution | Time (minutes) | Flow Rate (mL/min) |
|---|---|---|
| Type 1 Water | 5 | 400 |
| Type I Water | 5 | 400 |
| Type I Water | 5 | 400 |
| Type I Water | 5 | 400 |
| 2X PBS | 5 | 700 |
| 2X PBS | 5 | 700 |
| 2X PBS | 5 | 700 |
| Type 1 Water | 10 | 750 |

(continued)

| Solution | Time (minutes) | Flow Rate (mL/min) |
|---|---|---|
| 0.02% Trypsin/0.05% EDTA (flow rate increases over time without solution changes) | 60 | 1200 |
| | 60 | 1500 |
| | 60 | 1800 |
| Type 1 Water | 10 | 1900 |
| 2X PBS | 10 | 1950 |
| 3% Triton X-100/0.05% EDTA | 60 | 2000 |
| 3% Triton X-100/0.05% EDTA | 90 | 2100 |
| Type 1 Water | 10 | 2150 |
| 2X PBS | 10 | 2180 |
| 4% Deoxycholic Acid | 180 | 2200 |
| Type 1 Water | 5 | 2200 |
| Type 1 Water | 5 | 2200 |
| Type 1 Water | 5 | 2200 |
| 2X PBS | 5 | 2200 |
| 2X PBS | 5 | 2200 |
| 2X PBS | 5 | 2200 |
| 0.1% Peracetic Acid | 90 | 2200 |
| 1X PBS | 15 | 2200 |
| Type 1 Water | 5 | 2200 |
| Type 1 Water | 5 | 2200 |
| 1X PBS | 15 | 2200 |
| Type 1 Water | 5 | 2200 |
| Type 1 Water | 5 | 2200 |
| Non-recirculating deionized water | 15 | 2200 |

The final wash was performed with deionized water under non-recirculating flow from a separate reservoir. The left ventricle was dissected from the resulting decellularized tissue, cut into 3-4 mm sized pieces (discarding the chordae tendinae), frozen, and lyophilized until dry. The material was then delipidized by extraction with 2:1 chloroform:methanol (v/v) for 30 min with stirring. Solvent was drained and removed by three washes with 100% ethanol for 30 min each. The resulting product was rehydrated with 70% ethanol followed by 2 washes with deionized water for 30 min each followed by freezing and lyophilization. The material was then milled into a fine particulate using a Wiley Mini Mill (Thomas Scientific, Swedesboro, NJ) and filtered through a 40 mesh screen. Particulate ECM was enzymatically digested at a concentration of 20 mg ECM/mL using 2 mg/mL pepsin (Sigma) in 0.01 M HCl for 48 hours at room temperature with constant stirring. Prior to electrospinning, digested ECM was neutralized on ice with 1:10 of the digest volume of 0.1 M NaOH and 1:9 the digest volume of 10X PBS, and further diluted to a final ECM concentration of 15 mg/mL using 1X PBS.

2. Cardiac patch fabrication

Cardiac patches were fabricated by a modified two stream electrospinning process (Takanari K, et al., Abdominal wall reconstruction by a regionally distinct biocomposite of extracellular matrix digest and a biodegradable elastomer. Journal of tissue engineering and regenerative medicine. 2013:n/a-n/a) as illustrated in Figure 2B. A rotating, rastering steel cylinder of (D=114 mm) was utilized as a collecting target. Metal components utilized in patch fabrication and handling were autoclaved. Fiber deposition was concentrated on a well-defined area ($\pi DL$ where $L$=4.5cm, Figure 2C) by limiting the rastering span to 6 cm and by utilizing electrical insulating tape. PECUU was dissolved in hexafluoroisopropanol (12% w/v). For the ECM integrated cardiac patch, two distinct layers (Figures 2D and 2E) were created by sequential 20 min deposition periods. For the first, polymer-rich layer, the two concurrent depositing streams were 1) a PECUU solution at 20 mL/hr, from 10 cm, and 13 kV charge, and 2) a PBS solution at 1.35 mL/min, from 4 cm, and 8 kV charge. For the second, ECM-rich layer, identical parameters were utilized for the PECUU stream, while the second stream was the ECM solution described above at 1.35 mL/min, from 4 cm, and at a 13 kV charge. For the cardiac patches without ECM, one layer was fabricated by deposition from two streams for 40 min, using the same parameters as for the polymer-rich layer described above. The collecting mandrel voltage was kept at -4kV, while rotating at 750 rpm and translating at 0.15 cm/s for all patch fabrication processes. For ECM-integrated patches, scaffolds were then incubated at 37°C for 45 min to induce ECM solution gelification. Finally, cardiac patches were obtained by cutting disks of 6 mm diameter with a surgical punch. The stiffer direction was highlighted by cutting with a surgical blade one edge of the patch parallel to the mandrel circumferential direction. Each side of the cardiac patches were exposed to UV light for 30 min each prior to implantation, which was performed within 24 hours of fabrication. Thermally induced phase separation scaffolds utilized for pre-implant comparison were fabricated as described in (Fujimoto KL, et al., An Elastic, Biodegradable Cardiac Patch Induces Contractile Smooth Muscle and Improves Cardiac Remodeling and Function in Subacute Myocardial Infarction. Journal of the American College of Cardiology. 2007;49:2292-300).

3. Animal and surgical models

Adult female Lewis rats (200-250 g, Harlan Sprague Dawley, Indianapolis, Indiana) were utilized following animal care and surgical protocols in accordance with the National Institutes of Health guidelines for animal care and following protocol approval by the University of Pittsburgh IACUC. Myocardial infarction was induced by proximal left coronary artery ligation, as described in Fujimoto KL, et al., In Vivo Evaluation of a Porous, Elastic, Biodegradable Patch for Reconstructive Cardiac Procedures, The Annals of Thoracic Surgery, 2007;83:648-54; Fujimoto KL, et al., Placement of an Elastic Biodegradable Cardiac Patch on a Subacute Infarcted Heart Leads to Cellularization With Early Developmental Cardiomyocyte Characteristics, Journal of Cardiac Failure, 2012;18:585-95; Fujimoto KL, et al., An Elastic, Biodegradable Cardiac Patch Induces Contractile Smooth Muscle and Improves Cardiac Remodeling and Function in Subacute Myocardial Infarction, Journal of the American College of Cardiology, 2007;49:2292-300; and Hashizume R, et al., The effect of polymer degradation time on functional outcomes of temporary elastic patch support in ischemic cardiomyopathy, Biomaterials, 2013;34:7353-63. In brief, rats inhaled 3% isofluorane with 100 % oxygen followed by intubation and respiratory support provided by a rodent ventilator (683 Ventilator, Harvard Apparatus, Holliston, MA). The heart was accessed through a left thoracotomy, and then monitored by electrocardiography and tail cuff blood pressure. Ligation was performed with 7-0 polypropylene suture, ischemia conditions were verified by both ST-segment elevation and regional cyanosis. Finally, the incision was closed with a 4-0 continuous suture line. Two weeks after infarction, the animals were anesthetized and infarct size was estimated by echocardiography as the percentage of scar (akinetic or dyskinetic regions) and LV free wall areas. A total of 49 rats having infarcts larger than 25% of the LV free wall were randomized and divided into four groups: infarction control $n$=15, longitudinally oriented patch $n$=11, orthogonally oriented patch $n$=11, and ECM patch n=12. Six age-matched rats without coronary ligation or patch implantation served as a healthy control group. The animals in the MI group did not receive a second thoracotomy. The infarcted anterior LV wall was accessed through a left thoracotomy (Fujimoto KL, et al., Biodegradable Patch for Reconstructive Cardiac Procedures, The Annals of Thoracic Surgery, 2007;83:648-54; Fujimoto KL, et al., Placement of an Elastic Biodegradable Cardiac Patch on a Subacute Infarcted Heart Leads to Cellularization With Early Developmental Cardiomyocyte Characteristics, Journal of Cardiac Failure, 2012;18:585-95; Fujimoto KL, et al., An Elastic, Biodegradable Cardiac Patch Induces Contractile Smooth Muscle and Improves Cardiac Remodeling and Function in Subacute Myocardial Infarction, Journal of the American College of Cardiology, 2007;49:2292-300; and Hashizume R, et al., The effect of polymer degradation time on functional outcomes of temporary elastic patch support in ischemic cardiomyopathy, Biomaterials, 2013;34:7353-63), before affixing the patch, the epicardial surface was lightly scraped (removing both fibrotic tissue and remnant epicardium to a level <100 μm) at the patch implant site. Next, the patch was affixed on the anterior infarcted myocardium using 7-0 polypropylene with over and over peripheral continuous sutures.

4. Echocardiography

Echocardiographic assessment was performed on each animal at the time of ligation, prior to patch implantation (2 weeks post-infarction), as well as at 4 and 8 weeks after patch implantation. Rats were anesthetized with 1.25-1.5% isoflurane with 100% oxygen inhalation. Standard transthoracic echocardiography was performed using the Acuson Sequoia C256 system with a 13-MHz linear ultrasonic transducer (15L8; Acuson Corporation, Mountain View, California) in a phased-array format. B-mode measurements on the LV short axis view (papillary muscle level) were performed. The end-diastolic (EDA) and end-systolic (ESA) LV internal cavity areas were measured by tracing the endocardial border. The fractional area change (%FAC) was calculated as:

$$\%\mathrm{FAC} = \left[\frac{\mathrm{EDA} - \mathrm{ESA}}{\mathrm{EDA}}\right] \times 100\%.$$

All measurements were performed using OsiriX digital image processing application v.3.7.1.

5. Global mechanical compliance

Ex vivo mechanical global compliance testing was implemented to further characterize LV global mechanics (Fujimoto KL, et al., In Vivo Evaluation of a Porous, Elastic, Biodegradable Patch for Reconstructive Cardiac Procedures, The Annals of Thoracic Surgery, 2007;83:648-54). Explants were tested within 12 hours of animal sacrifice. A size 5 latex balloon, mounted on a flexible catheter (AD Instruments, Dunedin, New Zealand) was collapsed and introduced in the explanted LV through the aorta. The catheter was equipped with a Mikro-Cath™ (Millar Inc. Houston TX), and fed by water with a Ph2000 pump (Harvard Bioscience, Holliston, MA). Pressure and volume signals were acquired with a NISBC68 and a custom made script developed in LabVIEW 13.0 (National Instruments, Austin, TX). The adoption of a balloon allowed for a more precise pressure - volume characterization preventing fluid leakage from the heart LV and from the coronary bed. The LV volume variations were imposed by the pump while the same catheter measured the pressure. The tests (n≥6/group) were conducted in pressure-control mode under quasi-static conditions (0.25 mL/min). Five cycles of preconditioning, reaching a peak pressure of 200-250 mmHg, were performed to ensure proper contact between the endocardium and the balloon.

6. Multi-photon imaging

Multiphoton imaging was adopted on pre-implant patches as well as on control group (infarcted and healthy LV) samples and cardiac patch explants (n=5/group) to visualize de novo ECM structure, native collagen fibers (Takanari K, et al., Abdominal wall reconstruction by a regionally distinct biocomposite of extracellular matrix digest and a biodegradable elastomer, Journal of tissue engineering and regenerative medicine, 2013:n/a-n/a), and the remaining polymer fibers at the 8 week time point. An Olympus FV 1000 multi-photon microscope (Center Valley, PA) was utilized with excitation wavelength of 870 nm, laser power 5-7%, and sampling speed of 10 $\mu$s/pixel. Emission signals were acquired at $400 \pm 50$ nm for the de novo ECM and at $595 \pm 25$ nm for the scaffold fibers. Scanned volume/sample was $500 \times 500 \times 100$ $\mu$m$^3$ with steps of 10 $\mu$m. While collagen second harmonic generation signal did not require any amplification, scaffold fiber emission signals were augmented by adding a molecular probe. In brief, each patch explant (disk of ~6 mm diameter) was incubated with 50 mg of Red CMTPX (Molecular Probes) diluted in 5 mL of dimethyl sulfoxide, and 50 mL of distilled water (D' Amore A, et al., From single fiber to macro-level mechanics: A structural finite-element model for elastomeric fibrous biomaterials, Journal of the Mechanical Behavior of Biomedical Materials, 2014;39:146-61). ECM pre-implant patches were imaged from both the ECM-rich and polymer-rich sides. Image stacks were also analyzed quantitatively by a digital image processing to capture the level of collagen fiber alignment. A widely adopted orientation index (OI) was utilized as a metric for alignment (OI=1 parallel fibers, OI=0.5 isotropic fibers) (Takanari K, et al., Abdominal wall reconstruction by a regionally distinct biocomposite of extracellular matrix digest and a biodegradable elastomer, Journal of tissue engineering and regenerative medicine, 2013:n/a-n/a; D' Amore A, et al., From single fiber to macro-level mechanics: A structural finite-element model for elastomeric fibrous biomaterials, Journal of the Mechanical Behavior of Biomedical Materials, 2014;39:146-61; and Koch RG, et al., A custom image-based analysis tool for quantifying elastin and collagen micro-architecture in the wall of the human aorta from multi-photon microscopy, Journal of Biomechanics, 2014;47:935-43).

7. Scanning electron microscopy and scaffold surface characterization

For surface morphology characterization, pre-implant cardiac patch specimens (n=6/group) were sputter coated with Pd/Au and the polymer rich side was imaged (grayscale, 8-bit) using a standard SEM (JEOL JSM6330F). Seven images were selected from random locations of each sample. Finally, a previously developed custom made software enabled the quantification of: bulk porosity, fiber OI, node density (number of overlaying fibers/unit area), fiber mean diameter, and pore mean area (D'Amore A, et al., Characterization of the complete fiber network topology of planar fibrous tissues and scaffolds, Biomaterials, 2010, 31:5345-54).

8. Biaxial testing

Biaxial mechanical testing was performed on pre-implant scaffolds, control group samples and cardiac patch explants at 8 weeks (*n*≥5/group) (Sacks M., Biaxial Mechanical Evaluation of Planar Biological Materials, Journal of Elasticity, 2000, 61:199-246). Explants were tested within 12 hours of animal sacrifice. A $10 \times 10$ mm$^2$ sample centered on the cardiac patch was removed from the explanted hearts or simply cut from the pre-implant patch. This approach enabled the characterization of the patch area and the surrounding remodeled tissue. Tests were conducted in equi-membrane Lagrangian tension control reaching a peak value of 70 N/m (Takanari K, et al., Abdominal wall reconstruction by a regionally distinct biocomposite of extracellular matrix digest and a biodegradable elastomer, Journal of tissue engineering and regenerative medicine, 2013:n/a-n/a). This value was identified by testing healthy LV explants targeting membrane tensions capable of inducing physiologically relevant stretches (1.0 - 1.20) (Fomovsky GM, et al., Evolution of scar structure, mechanics, and ventricular function after myocardial infarction in the rat, 2010). Four fiducial markers were affixed in a square configuration and were used to calculate the deformation gradient tensor. After preconditioning, samples were tested at room temperature in PBS for 10 cycles of 30s each. The free-floating configuration after pre-conditioning was utilized as the reference for post-processing.

9. Histology and immunofluorescence

Whole heart explants (*n*=6/group) were fixed in 2% paraformaldehyde for 3 hours at 4°C, embedded with OCT compound (Tissue-Tek, Torrance, CA) and frozen at -80 °C until sectioning at 10 $\mu$m thickness in the LV transverse direction, mounting on glass slides and staining with Masson's trichrome. In order to verify the quality of the decellularization process, decellularized porcine LV samples utilized to generate the ECM gel were processed with the same protocol utilized for rat heart explants and stained with DAPI, hematoxylin and eosin (Figure 3). For immunofluorescence assessment 6 independent samples for each group were evaluated for each staining type. Sections were fixed in 4% paraformaldehyde and blocked with 10% goat serum in 0.2% Triton - 1 X PBS solution for 2 hours at room temperature. To quantify macrophage infiltration, sections were immune-labeled with rabbit primary antibody against CD68 (ab125212; 1:100, Abcam) followed by goat anti-rabbit secondary antibody Alexa Fluor® 488 (A-1108; 1:1500, Thermo Fisher Scientific). In addition, a mouse primary antibody against CD163 (sc-58965; 1:100, Santa Cruz) was utilized as a macrophage phenotype M2 specific marker, and coupled with the secondary anti-mouse antibody Alexa Fluor® 459 (A-11032; 1/200, Invitrogen). Mouse primary antibody against CD31 (ab64543, 1:100, Abcam) and rabbit primary antibody against $\alpha$SMA (Ab5694, 1:100, Abcam) were utilized to quantify conductance blood vessels, with secondary antibodies of anti-rabbit Alexa Fluor® 594 (A21207; 1/1000, Thermo Fisher Scientific) and biotin-streptadivin Alexa Fluor® 488 (BA-2001, 1:200, Thermo Fisher Scientific - 532354, 1:150, Invitrogen). In order to further assess vessel formation, pericytes and their association with vascular structures were studied by von Willebrand factor (vWf, V2700-01C, 1:300, US Biological) and NG2 (Ab5320, 1/150, Millipore) co-staining with secondary antibody for NG2 of anti-rabbit Alexa Fluor 488 at 1:200 (Sicari BM, et al., An Acellular Biologic Scaffold Promotes Skeletal Muscle Formation in Mice and Humans with Volumetric Muscle Loss. Science Translational Medicine, 2014, 6:234ra58-ra58, and Crisan M, et al., A Perivascular Origin for Mesenchymal Stem Cells in Multiple Human Organs, Cell Stem Cell, 2008, 3:301-13)

$\alpha$SMA and its related secondary antibody were also adopted to quantify myofibroblast recruitment (Amir G, et al., Evaluation of a Peritoneal-Generated Cardiac Patch in a Rat Model of Heterotopic Heart Transplantation, Cell Transplantation, 2009;18:275-82) or interstitial fibroblast phenotypic translation (Lakshmanan R, et al., Living cardiac patch: the elixir for cardiac regeneration, Expert Opinion on Biological Therapy, 2012, 12:1623-40). Finally, sections were incubated with DAPI mounting medium to provide nuclear staining, (5 $\mu$L/section, DAPI H-1200, Vectashield). Negative controls were obtained from sections that were stained with secondary antibody only (Fujimoto KL, et al., In Vivo Evaluation of a Porous, Elastic, Biodegradable Patch for Reconstructive Cardiac Procedures, The Annals of Thoracic Surgery, 2007, 83:648-54, and Hashizume R, et al., The effect of polymer degradation time on functional outcomes of temporary elastic patch support in ischemic cardiomyopathy, Biomaterials, 2013, 34:7353-63).

Multispectral epifluorescent images were acquired using a Nikon Eclipse 6600 microscope (Nikon Corporation), with spectral unmixing to remove autofluorescence performed using Nuance 3.0.2 software (Caliper Life Science Inc.) (Faulk DM, et al., ECM hydrogel coating mitigates the chronic inflammatory response to polypropylene mesh, Biomaterials, 2014, 35:8585-95). For each slide eleven fields were acquired at 200X (two images in proximity to the suture areas, three images inside, three images above and three images below the patch region). CD68 and CD163 positive cells were identified with custom-made software developed in Matlab. In brief, Otsu's binary segmentation was operated on marker-specific channels to remove residual noise and further identify positive pixels, the cell number was given by the ratio between the mean cell pixel size and total number of marker positive pixels (D'Amore A, et al., Characterization of the complete fiber network topology of planar fibrous tissues and scaffolds, Biomaterials, 2010, 31:5345-54). CD163+/CD68+ ratio was then calculated. The same image segmentation process enabled the identification of $\alpha$SMA positive pixels and cell nuclear aspect ratio (NAR) defined as the ratio between the major and minor axes of the nuclei (D'Amore A, et al., From single fiber to macro-level mechanics: A structural finite-element model for elastomeric fibrous biomaterials, Journal of the Mechanical Behavior of Biomedical Materials, 2014;39:146-61). Tubular structures positive for both CD31 and $\alpha$SMA were identified as mature vessels and further classified into: a) arterioles and post-capillary veins, b) small arteries and venules, c) small veins, d) arteries, and e) veins based on size, shape, and tunica media/tunica intima ($\alpha$SMA/CD31) ratio

(Table 2, as shown below) (Wiedeman MP, Dimensions of Blood Vessels from Distributing Artery to Collecting Vein. Circulation Research, 1963, 12:375-8).

Table 2: Blood vessel classification

|  | Size [$\mu$m] | Rounded Shape | CD31/$\alpha$SMA |
|---|---|---|---|
| **Arterioles and post capillary veins** | $5 \leq D \leq 10$ | NA | NA |
| **Small artery and venule** | $10 \leq D \leq 20$ | NA | NA |
| **Small veins** | $20 \leq D \leq 30$ | NA | Comparable tunica media and intima |
| **Artery** | $D \geq 50$ | YES | Prominent media |
| **Vein** | $D \geq 75$ | NA | Comparable tunica media and intima |

10. Quantification of muscle, scar area, and LV anterior wall thickness

LV cross-sections stained with Masson's trichrome were utilized to quantify muscle, scaffold/newly formed tissue, scar area and wall thickness (Hashizume R, et al., The effect of polymer degradation time on functional outcomes of temporary elastic patch support in ischemic cardiomyopathy, Biomaterials, 2013;34:7353-63; Takanari K, et al., Abdominal wall reconstruction by a regionally distinct biocomposite of extracellular matrix digest and a biodegradable elastomer, Journal of tissue engineering and regenerative medicine, 2013:n/a-n/a; and Cassino TR, et al., Mechanical Loading of Stem Cells for Improvement of Transplantation Outcome in a Model of Acute Myocardial Infarction: The Role of Loading History, Tissue Engineering Part A, 2012, 18:1101-8). Scar and muscle areas pixels were measured on ImageJ (n=6/group) using computer-based planimetry. In order to eliminate the variability induced by the different heart sizes or due to differences in the sectioning plane, the following process was utilized: a digital frame of constant size was applied to each sample to extract scar and muscle areas, as well as the total area occupied by the explants. Scar, scaffold/new tissue, and muscle indices were defined as the ratio between the scar or muscle area and the total area occupied by the explants (Dvir T, et al., Prevascularization of cardiac patch on the omentum improves its therapeutic outcome, Proceedings of the National Academy of Sciences, 2009, 106:14990-5). LV anterior wall thickness was calculated as: scar area/[(epicardial circumference + endocardial circumference)/2] (Hashizume R, et al., The effect of polymer degradation time on functional outcomes of temporary elastic patch support in ischemic cardiomyopathy, Biomaterials, 2013, 34:7353-63).

11. Statistical analyses

Statistical analyses were performed using Sigma plot (Systat Software Inc., Chicago, IL, USA). Normal distribution of data was verified by the Kolmogorov-Smirnov test. One-way analysis of variance (ANOVA) followed by Tukey-Kramer multiple comparison testing was utilized for comparison of multiple samples. When the data distribution was not normally distributed, the Krusal-Wallis non-parametric multiple-comparison test was employed. In order, to compare the biaxial mechanics of different groups, one-way ANOVA was applied to compare the maximum stretch for each sample. Results are presented as mean $\pm$ standard error of the mean and differences were considered to be statistically significant at p<0.05.

*Results:*

1. Material characterization

Successful completion of the decellularization process was confirmed visually (Figure 3A-B), histologically (Figure 3C-F) and via immunofluorescence imaging (Figure 3G-H) (Remlinger NT, et al., Procedure for decellularization of porcine heart by retrograde coronary perfusion, J Vis Exp 2012, p. e50059, and Faulk DM, et al., ECM hydrogel coating mitigates the chronic inflammatory response to polypropylene mesh, Biomaterials, 2014, 35:8585-95). Left ventricle cross sections (Figure 3B) qualitatively showed transmural consistency in the decellularization process.

Bi-layered patch structure was also verified visually (Figure 2D), histologically (Figure 2E) and with multi-photon microscopy (Figure 4A-B). Morphological evidence, as well as physical handling of the specimens, confirmed structural continuity between the two layers with no signs of delamination. Structure-function relationships were further characterized by biaxial testing (Figure 4C). Similarly, pre-implant mechanics are presented in Figure 4D, with scaffold types tested including: thermally induced phase separation (TIPS), patch reproducing Fujimoto *et al.* (Fujimoto KL, et al., In Vivo Evaluation of a Porous, Elastic, Biodegradable Patch for Reconstructive Cardiac Procedures, The Annals of Thoracic Surgery, 2007, 83:648-54), orthogonally aligned PECUU cardiac patch (Ortho) and the bi-layered ECM cardiac patch. While all these materials shared comparable elastic moduli, only the orthogonally and longitudinally oriented groups had a statistically significant level of anisotropy, showing one direction significantly more compliant than the other (p<0.05).

Pre-implant material surface visualization by scanning electron microscopy and image analysis is presented in Figure 5.

2. ESA, EDA and %FAC by echocardiography

As expected, echocardiographic assessment showed for each time point a higher ESA, EDA and lower %FAC for the infarcted control animals when compared to the healthy control group (Figure 6). However, neither the orthogonally or longitudinally oriented patches had a significant effect on either functional parameter versus the infarcted control group. In contrast, the ECM group had a beneficial functional effect, with a lower ESA, EDA and higher %FAC than the infarct control group, albeit still significantly less functional than the healthy controls. In order to further assess ventricle remodeling, representative echocardiograpic images in systole and diastole at 0, 4 and 8 week time-points are presented in Figure 6A. Endocardial side (red dotted edges - in original) and epicardial side (white dotted edges - in original) are highlighted in systole at the 1 and 8 week time-points, showing both the efficacy of the adopted infarction model and the beneficial effect of ECM patching as reflected in Figure 6B.

3. Histology, muscle, scar areas, and LV anterior wall thickness

LV anterior wall thickness (n=6/group) was higher in the ECM patch treated group than in the infarcted control, while not statistically varying from the healthy control group (Figure 7). Muscle, scaffold/new tissue and scar area indices based on quantitative measurements performed on 8 weeks tissue sections stained with Masson's trichrome are provided in Figure 8. Consistent with qualitative observation of the histology data, the healthy control group had a scar index equal to 0 while, the ECM group had a lower scar index than the orthogonal and the MI control groups. Similarly, the healthy control and ECM groups had a cumulative muscle plus scaffold/new tissue index higher than the MI control.

4. Multi-photon imaging

Multi photon analysis was performed to visualize collagen and scaffold fibers and therefore to visualize de novo ECM deposition and evidence of scaffold degradation. In Figure 9 native and de-novo collagen second harmonic generation (top row) were visualized together with residual scaffold components (middle row). The bottom row of images shows enlarged regions of interest from the middle row, together with a schematic of the putative fiber network.

In order to associate fiber-orientation metrics to the qualitative analysis of the forming ECM, de novo collagen fiber alignment has been quantified and compared to the aspect ratio for cell nuclei in Figure 10. The OI along the circumferential direction and NAR were higher for the orthogonal group when compared to the longitudinal end ECM groups.

5. Global mechanical compliance

LV pressure - volume relationships (Figure 11) were characterized *ex vivo* ($n \geq 6$/group) to quantify, under quasi-static conditions, the effect of patch treatment on global mechanical compliance. All the patch treated groups significantly stiffened the LV versus the infarct control. This is in contrast to the echocardiographically measured EDA, where only the ECM group differed from the infarct control (Figure 6).

6. Biaxial testing

Equi-membrane tension biaxial testing (Figure 12) was performed to characterize the mechanics of explanted tissue in the region of the infarct and patching ($n \geq 5$/group) at 8 weeks. The longitudinal and ECM groups explants showed an increase in stiffness over the circumferential direction when compared to the pre-implant configuration. Conversely, the orthogonal group showed an increase in stiffness in the longitudinal direction. All the patched groups showed a significantly stiffer response than the MI group over the longitudinal direction.

7. Immunofluorescence

Comparison between the longitudinal and orthogonal groups suggested that the patch mechanics had no impact on $\alpha$SMA expression (Figure 13). Host cell infiltration was higher in ECM explants as qualitatively suggested by Masson's trichrome sections (Figure 8) and as confirmed by counting the number of DAPI (blue in original) stained nuclei (Figure 14 A-D) within the patch region ($n \geq 5$/group) than for the longitudinal group. In spite of this higher number of cells infiltrating the patch, the ECM group showed a lower number of CD68 (green) positive cells (Figure 14 E) and a higher CD 68+/CD163+ ratio (CD163 positive cells are shown in red). Endocardium zone (Endo) and cardiac patch (CP) borders were highlighted in white. The top row provides representative pictures of the CP-Endo transition zones whereas the bottom row provides examples of the zones at the edges of the CP.

Quantification of conductance vessel number and type, as quantified by CD31 and $\alpha$SMA co-localization, associated with the patch area (Figure 15) showed a higher number of vessels/mm² for the ECM patch when compared with the orthogonal group at 8 weeks.

Immunofluorescence analysis for vessel quantification was further supported by high magnification Masson's trichrome staining (Figure 16).

Angiogenesis was further investigated by vWf and NG2 co-staining and the co-localization or the presence of vWf+ structures in proximity to NG2+ cells (Figure 17) inside the cardiac patch and underneath the treated areas at 8 weeks.

## Claims

1. A cardiac patch for use in a method of treating damage or a defect in a tissue in a patient, wherein the cardiac patch comprises:

   a first layer comprising a network of synthetic, biocompatible, bioerodible fibers and an aqueous solution dispersed throughout the network of synthetic, biocompatible, bioerodible fibers of the first layer, and
   a second layer comprising a network of synthetic, biocompatible, bioerodible fibers, attached to the first layer, and comprising acid protease-solubilized ECM dispersed throughout the network of synthetic, biocompatible fibers of the second layer,
   wherein the fibers of the first layer are arranged anisotropically,
   wherein the layers are fibrous electrospun layers, and the method comprises implanting said cardiac patch at or adjacent to the tissue damage or defect such that the second layer contacts the damaged or defective cardiac or cardiovascular tissue.

2. The cardiac patch for use in a method of claim 1, wherein the ECM material is solubilized heart ECM (cECM).

3. The cardiac patch for use in a method of claim 2, wherein the acid protease-solubilized ECM is prepared by solubilizing devitalized and/or decellularized heart tissue with an acid protease in an acidic solution and neutralizing the acidic solution, e.g. to pH 7.2-7.8, e.g. 7.4, and optionally is not dialyzed prior to solubilization.

4. The cardiac patch for use in a method of claim 1, wherein the synthetic, biocompatible fibers of the second layer are the same as the synthetic, biocompatible fibers of the first layer.

5. The cardiac patch for use in a method of claim 1, wherein the aqueous solution is saline, phosphate-buffered saline, or serum-free cell culture media.

6. The cardiac patch for use in a method of any one of claims 1 to 5, wherein the synthetic, biocompatible polymer of the first layer and/or the second layer comprises one or more of:

   a) a poly(ester urethane) urea (PEUU); a poly(ether ester urethane)urea (PEEUU); a poly(ester carbonate) urethane urea (PECUU); a poly(carbonate)urethane urea (PCUU) elastomer; or
   b) comprises monomers derived from alpha-hydroxy acids including, without limitation: polylactide, poly(lactide-co-glycolide), poly(L-lactide-co-caprolactone), polyglycolic acid, poly(dl-lactide-co-glycolide), poly(l-lactide-co-dl-lactide); monomers derived from esters including polyhydroxybutyrate, polyhydroxyvalerate, polydioxanone and polyglactin; monomers derived from lactones including polycaprolactone; monomers derived from carbonates including polycarbonate, polyglyconate, poly(glycolide-co-trimethylene carbonate), poly(glycolide-co-trimethylene carbonate-co-dioxanone); and monomers joined through urethane linkages, including polyurethane and poly(ester urethane) urea elastomers.

7. The cardiac patch for use in a method of any one of claims 1 to 6, wherein

   a) the damage or defect is in a left ventricular region of a heart;

b) the damage or defect in the cardiac or cardiovascular tissue is damage resulting from a myocardial infarction;
c) the damage or defect in the cardiac or cardiovascular tissue is a deficiency resulting from a congenital defect;
d) the damage or defect in the cardiac or cardiovascular tissue is a right ventricular outflow tract of a heart;
e) the damage or defect is in a heart valve; or
f) the damage or defect is in the aorta or vena cava.

**Patentansprüche**

1. Herzpflaster zur Verwendung bei einem Verfahren zur Behandlung einer Schädigung oder eines Defekts in einem Gewebe eines Patienten, wobei das Herzpflaster umfasst:

   eine erste Schicht, die ein Netzwerk aus synthetischen, biokompatiblen, bioerodierbaren Fasern und eine wässrige Lösung umfasst, die in dem Netzwerk aus synthetischen, biokompatiblen, bioerodierbaren Fasern der ersten Schicht dispergiert ist, und
   eine zweite Schicht, die ein Netzwerk aus synthetischen, biokompatiblen, bioerodierbaren Fasern umfasst, die an der ersten Schicht befestigt ist und eine durch Säureproteasen lösliche ECM umfasst, die in dem Netzwerk aus synthetischen, biokompatiblen Fasern der zweiten Schicht dispergiert ist,
   wobei die Fasern der ersten Schicht anisotrop angeordnet sind,
   wobei die Schichten faserige elektrogesponnene Schichten sind, und
   das Verfahren das Implantieren des Herzpflasters an oder neben der Gewebeschädigung oder dem Gewebedefekt umfasst, so dass die zweite Schicht das beschädigte oder defekte Herz- oder Herz-Kreislauf-Gewebe kontaktiert.

2. Herzpflaster zur Verwendung in einem Verfahren nach Anspruch 1, wobei das ECM-Material solubilisierte Herz-ECM (cECM) ist.

3. Herzpflaster zur Verwendung in einem Verfahren nach Anspruch 2, wobei die mit einer sauren Protease solubilisierte ECM hergestellt wird, indem devitalisiertes und/oder dezellularisiertes Herzgewebe mit einer sauren Protease in einer sauren Lösung solubilisiert und die saure Lösung neutralisiert wird, z. B. auf pH 7,2-7,8, z. B. 7,4, und optional vor der Solubilisierung nicht dialysiert wird.

4. Herzpflaster zur Verwendung in einem Verfahren nach Anspruch 1, wobei die synthetischen, biokompatiblen Fasern der zweiten Schicht dieselben sind wie die synthetischen, biokompatiblen Fasern der ersten Schicht.

5. Herzpflaster zur Verwendung in einem Verfahren nach Anspruch 1, wobei die wässrige Lösung Kochsalzlösung, phosphatgepufferte Kochsalzlösung oder serumfreies Zellkulturmedium ist.

6. Herzpflaster zur Verwendung in einem Verfahren nach einem der Ansprüche 1 bis 5, wobei das synthetische, biokompatible Polymer der ersten Schicht und/oder der zweiten Schicht eines oder mehrere der folgenden Elemente umfasst:

   a) einen Poly(esterurethan)harnstoff (PEUU); einen Poly(etheresterurethan)harnstoff (PEEUU); einen Poly(estercarbonat)urethanharnstoff (PECUU); ein Poly(carbonat)urethanharnstoff (PCUU)-Elastomer; oder
   b) Monomere, die von Alpha-Hydroxysäuren abgeleitet sind, einschließlich, ohne Einschränkung: Polylactid, Poly(lactid-co-glycolid), Poly(L-lactid-co-caprolacton), Polyglykolsäure, Poly(dl-lactid-co-glycolid), Poly(l-lactid-co-dl-lactid); Monomere, die von Estern abgeleitet sind, einschließlich Polyhydroxybutyrat, Polyhydroxyvalerat, Polydioxanon und Polyglactin; Monomere, die von Lactonen abgeleitet sind, einschließlich Polycaprolacton; Monomere, die von Carbonaten abgeleitet sind, einschließlich Polycarbonat, Polyglyconat, Poly(glycolid-co-trimethylencarbonat), Poly(glycolid-co-trimethylencarbonat-co-dioxanon); und Monomere, die über Urethanbindungen verbunden sind, einschließlich Polyurethan und Poly(esterurethan)harnstoff-Elastomere.

7. Herzpflaster zur Verwendung in einem Verfahren nach einem der Ansprüche 1 bis 6, wobei

   a) die Schädigung oder der Defekt in einem Bereich der linken Herzkammer liegt;
   b) die Schädigung oder der Defekt im Herz- oder Herz-Kreislauf-Gewebe eine Schädigung infolge eines Myokardinfarkts ist;
   c) die Schädigung oder der Defekt im Herz- oder Herz-Kreislauf-Gewebe eine durch einen angeborenen Defekt bedingte Fehlbildung ist;

d) die Schädigung oder der Defekt im Herz- oder Herz-Kreislauf-Gewebe ein rechtsventrikulärer Ausflusstrakt eines Herzens ist;

e) die Schädigung oder der Defekt in einer Herzklappe liegt; oder

f) die Schädigung oder der Defekt in der Aorta oder der Hohlvene liegt.

**Revendications**

1. Patch cardiaque destiné à être utilisé dans un procédé de traitement d'une lésion ou d'un défaut dans un tissu chez un patient, dans lequel le patch cardiaque comprend :

   une première couche comprenant un réseau de fibres synthétiques, biocompatibles et bioérodables et une solution aqueuse dispersée dans tout le réseau de fibres synthétiques, biocompatibles et bioérodables de la première couche, et
   une deuxième couche comprenant un réseau de fibres synthétiques, biocompatibles et bioérodables, fixées à la première couche, et comprenant une MEC solubilisée par une protéase acide dispersée dans tout le réseau de fibres synthétiques biocompatibles de la deuxième couche,
   dans lequel les fibres de la première couche sont disposées de manière anisotrope,
   dans lequel les couches sont des couches électrofilées fibreuses, et
   le procédé comprend l'implantation dudit patch cardiaque au niveau ou à proximité de la lésion ou du défaut tissulaire de telle sorte que la deuxième couche entre en contact avec le tissu cardiaque ou cardiovasculaire lésé ou défectueux.

2. Patch cardiaque destiné à être utilisé dans un procédé selon la revendication 1, dans lequel le matériau MEC est de la MEC cardiaque solubilisée (cMEC).

3. Patch cardiaque destiné à être utilisé dans un procédé selon la revendication 2, dans lequel la MEC solubilisée par une protéase acide est préparée en solubilisant du tissu cardiaque dévitalisé et/ou décellularisé avec une protéase acide dans une solution acide et en neutralisant la solution acide, par exemple à un pH de 7,2 à 7,8, par exemple 7,4, et n'est éventuellement pas dialysée avant la solubilisation.

4. Patch cardiaque destiné à être utilisé dans un procédé selon la revendication 1, dans lequel les fibres synthétiques biocompatibles de la deuxième couche sont les mêmes que les fibres synthétiques biocompatibles de la première couche.

5. Patch cardiaque destiné à être utilisé dans un procédé selon la revendication 1, dans lequel la solution aqueuse est une solution saline, une solution saline tamponnée au phosphate ou un milieu de culture cellulaire sans sérum.

6. Patch cardiaque destiné à être utilisé dans un procédé selon l'une quelconque des revendications 1 à 5, dans lequel le polymère synthétique biocompatible de la première couche et/ou de la deuxième couche comprend un ou plusieurs des composés suivants :

   a) un poly(ester uréthane)urée (PEUU) ; un poly(éther ester uréthane)urée (PEEUU) ; un poly(ester carbonate) uréthane urée (PECUU) ; un élastomère poly(carbonate)uréthane urée (PCUU) ; ou
   b) comprend des monomères dérivés d'acides alpha-hydroxy, y compris, sans limitation : polylactide, poly(lactide-co-glycolide), poly(L-lactide-co-caprolactone), acide polyglycolique, poly(dl-lactide-co-glycolide), poly(1-lactide-co-dl-lactide) ; des monomères dérivés d'esters, notamment le polyhydroxybutyrate, le polyhydroxyvalérate, le polydioxanone et la polyglactine ; des monomères dérivés de lactones, notamment le polycaprolactone ; des monomères dérivés de carbonates, notamment le polycarbonate, le polyglyconate, le poly(glycolide-co-triméthylène carbonate), le poly(glycolide-co-triméthylène carbonate-co-dioxanone) ; et des monomères liés par des liaisons uréthane, notamment le polyuréthane et les élastomères de poly(ester uréthane) urée.

7. Patch cardiaque destiné à être utilisé dans un procédé selon l'une quelconque des revendications 1 à 6, dans lequel

   a) la lésion ou le défaut se trouve dans une région ventriculaire gauche du cœur ;
   b) la lésion ou le défaut dans le tissu cardiaque ou cardiovasculaire est une lésion résultant d'un infarctus du myocarde ;

c) la lésion ou le défaut dans le tissu cardiaque ou cardiovasculaire est une déficience résultant d'une malformation congénitale ;

d) la lésion ou le défaut dans le tissu cardiaque ou cardiovasculaire est une voie d'éjection ventriculaire droite d'un cœur ;

e) la lésion ou le défaut se trouve dans une valve cardiaque ; ou

f) la lésion ou le défaut se trouve dans l'aorte ou la veine cave.

**20** **10**

**30**

**35**

**Fig 1A**

**45** **20** **10**

**30**

**35** **40**

**Fig. 1B**

**Fig. 2**

*Fig. 3*

**Fig. 4A**

Fig. 4B

EP 3 347 063 B1

**A** Polymer side pores

**B** Polymer side fiber network

1 µm

**C** Pre-implant macro and micro geometry characterization

| | |
|---|---|
| Porosity | 87% |
| OI ( along CD ) | 0.57 ± 0.034 |
| Node density | 0.077 ± 0.008 node/µm$^2$ |
| Fiber diameter | 1.32 ± 0.05 µm |
| Pore mean area | 10 ± 1.2 µm$^2$ |
| Pore size | 3.2 ± 1.1 µm |
| Patch Thickness Long - Ortho | Total: 339 ± 1.7 µm |
| Patch Thickness ECM | Total: 258 ± 1.8 µm; ECM: 134 µm; Polymer: 124 µm; |
| Patch diameter | 6 mm |

**D** Polymer side

10 µm

**Fig. 5**

*Fig. 6A*

Fig. 6B

Fig. 7

EP 3 347 063 B1

Fig. 8

*Fig. 9*

Fig. 10A

**Fig. 10B**

**Fig. 11**

Fig. 12

*Fig. 13*

**Fig. 14**

Fig. 15

*Fig. 16*

**Fig. 17**

## REFERENCES CITED IN THE DESCRIPTION

### Patent documents cited in the description

- US 62216728
- US 4902508 A
- US 4956178 A
- US 5281422 A
- US 5352463 A
- US 5372821 A
- US 5554389 A
- US 5573784 A
- US 5645860 A
- US 5771969 A
- US 5753267 A
- US 5762966 A
- US 5866414 A
- US 6099567 A
- US 6485723 B
- US 6576265 B
- US 6579538 B
- US 6696270 B
- US 6783776 B
- US 6793939 B
- US 6849273 B
- US 6852339 B
- US 6861074 B
- US 6887495 B
- US 6890562 B
- US 6890563 B
- US 6890564 B
- US 6893666 B
- US 2008260831 A
- US 5216115 A
- US 20110082545 A

### Non-patent literature cited in the description

- **GUOXU ZHAO et al.** Recent Advances in Electrospun Nanofibrous Scaffolds for Cardiac Tissue Engineering. *Adv. Funct. Mat.*, 2015, vol. 25, 5726-5738
- **STANKUS et al.** Hybrid nanofibrous scaffolds from electrospinning of a synthetic biodegradable elastomer and urinary bladder matrix. *J Biomater. Sci. Polym. Ed.*, 2008, vol. 19 (5), 635-652
- **HONG et al.** Tailoring the degradation kinetics of poly(ester carbonate urethane)urea thermoplastic elastomers for tissue engineering scaffolds Biomaterials. *Biomaterials*, 2010, vol. 31, 4249-4258
- **FIORDELISO, J et al.** Design, synthesis, and preliminary characterization of tyrosine-containing polyarylates: new biomaterials for medical applications. *J Biomater Sci Polym Ed. 1994*, 1994, vol. 5 (6), 497-510
- **HUANG, X et al.** A library of L-tyrosine-derived biodegradable polyarylates for potential biomaterial applications, part I: synthesis, characterization and accelerated hydrolytic degradation. *J Biomater Sci Polym Ed. 2009*, 2009, vol. 20 (7-8), 935-55
- **BOURKE, SL et al.** Polymers derived from the amino acid L-tyrosine: polycarbonates, polyarylates and copolymers with poly(ethylene glycol). *Adv Drug Deliv Rev*, 25 April 2003, vol. 55 (4), 447-66
- **CHAPPELOW, AV et al.** Neovascular age-related macular degeneration: potential therapies. *Drugs*, 2008, vol. 68 (8), 1029-36
- **BARAKAT MR et al.** VEGF inhibitors for the treatment of neovascular age-related macular degeneration. *Expert Opin Investig Drugs*, May 2009, vol. 18 (5), 637-46
- **XU C.Y.** ; **INAI R** ; **KOTAKI M** ; **RAMAKRISHNA S**. Aligned biodegradable nanofibrous structure: a potential for blood vessel engineering. *Biomaterials*, 2004, vol. 25, 877-86
- **LEE C.H.** ; **SHIN H.J.** ; **CHO I.H.** ; **KANG Y.M.** ; **KIM I.A.** ; **PARK K.D.** ; **SHIN, J.W.** Nanofiber alignment and direction of mechanical strain affect the ECM production of human ACL fibroblast. *Biomaterials*, 2005, vol. 26, 1261-1270
- **HONG Y et al.** Tailoring the degradation kinetics of poly(ester carbonate urethane)urea thermoplastic elastomers for tissue engineering scaffolds. *Biomaterials*, 2010, vol. 31, 4249-58
- *J Vis Exp*, 2012, e50059
- **TAKANARI K et al.** Abdominal wall reconstruction by a regionally distinct biocomposite of extracellular matrix digest and a biodegradable elastomer. *Journal of tissue engineering and regenerative medicine*, 2013
- **FUJIMOTO KL et al.** An Elastic, Biodegradable Cardiac Patch Induces Contractile Smooth Muscle and Improves Cardiac Remodeling and Function in Subacute Myocardial Infarction. *Journal of the American College of Cardiology*, 2007, vol. 49, 2292-300

- **FUJIMOTO KL et al.** In Vivo Evaluation of a Porous, Elastic, Biodegradable Patch for Reconstructive Cardiac Procedures. *The Annals of Thoracic Surgery*, 2007, vol. 83, 648-54
- **FUJIMOTO KL et al.** Placement of an Elastic Biodegradable Cardiac Patch on a Subacute Infarcted Heart Leads to Cellularization With Early Developmental Cardiomyocyte Characteristics. *Journal of Cardiac Failure*, 2012, vol. 18, 585-95
- **HASHIZUME R et al.** The effect of polymer degradation time on functional outcomes of temporary elastic patch support in ischemic cardiomyopathy. *Biomaterials*, 2013, vol. 34, 7353-63
- **FUJIMOTO KL et al.** Biodegradable Patch for Reconstructive Cardiac Procedures. *The Annals of Thoracic Surgery*, 2007, vol. 83, 648-54
- **D' AMORE A et al.** From single fiber to macro-level mechanics: A structural finite-element model for elastomeric fibrous biomaterials. *Journal of the Mechanical Behavior of Biomedical Materials*, 2014, vol. 39, 146-61
- **KOCH RG et al.** A custom image-based analysis tool for quantifying elastin and collagen micro-architecture in the wall of the human aorta from multi-photon microscopy. *Journal of Biomechanics*, 2014, vol. 47, 935-43
- **D'AMORE A et al.** Characterization of the complete fiber network topology of planar fibrous tissues and scaffolds. *Biomaterials*, 2010, vol. 31, 5345-54
- **SACKS M**. Biaxial Mechanical Evaluation of Planar Biological Materials. *Journal of Elasticity*, 2000, vol. 61, 199-246
- **FOMOVSKY GM et al.** *Evolution of scar structure, mechanics, and ventricular function after myocardial infarction in the rat*, 2010
- **SICARI BM et al.** An Acellular Biologic Scaffold Promotes Skeletal Muscle Formation in Mice and Humans with Volumetric Muscle Loss. *Science Translational Medicine*, 2014, vol. 6, 234ra58-ra58
- **CRISAN M et al.** A Perivascular Origin for Mesenchymal Stem Cells in Multiple Human Organs. *Cell Stem Cell*, 2008, vol. 3, 301-13
- **AMIR G et al.** Evaluation of a Peritoneal-Generated Cardiac Patch in a Rat Model of Heterotopic Heart Transplantation. *Cell Transplantation*, 2009, vol. 18, 275-82
- **LAKSHMANAN R et al.** Living cardiac patch: the elixir for cardiac regeneration. *Expert Opinion on Biological Therapy*, 2012, vol. 12, 1623-40
- **FAULK DM et al.** ECM hydrogel coating mitigates the chronic inflammatory response to polypropylene mesh. *Biomaterials*, 2014, vol. 35, 8585-95
- **D'AMORE A et al.** From single fiber to macro-level mechanics: A structural finite-element model for elastomeric fibrous biomaterials. *Journal of the Mechanical Behavior of Biomedical Materials*, 2014, vol. 39, 146-61
- **WIEDEMAN MP**. Dimensions of Blood Vessels from Distributing Artery to Collecting Vein. *Circulation Research*, 1963, vol. 12, 375-8
- **CASSINO TR et al.** Mechanical Loading of Stem Cells for Improvement of Transplantation Outcome in a Model of Acute Myocardial Infarction: The Role of Loading History. *Tissue Engineering Part A*, 2012, vol. 18, 1101-8
- **DVIR T et al.** Prevascularization of cardiac patch on the omentum improves its therapeutic outcome. *Proceedings of the National Academy of Sciences*, 2009, vol. 106, 14990-5
- **REMLINGER NT et al.** Procedure for decellularization of porcine heart by retrograde coronary perfusion. *J Vis Exp*, 2012, e50059